# EUROPEAN PATENT SPECIFICATION

(11) **EP 4 121 117 B1**
(45) Date of publication and mention of the grant of the patent: **08.10.2025**
(21) Application number: 21770606.8
(22) Date of filing: 17.03.2021
(51) Int. Cl.: A61K 47/62, A61P 25/00, A61K 9/107

(54) **DESIGNER EXTRACELLULAR VESICLES FOR TREATING EXCITOTOXICITY**
DESIGNER-EXTRAZELLULÄRE VESIKEL ZUR BEHANDLUNG VON EXZITOTOXIZITÄT
VÉSICULES EXTRACELLULAIRES DE CONCEPTION POUR LE TRAITEMENT DE L'EXCITOTOXICITÉ

(30) Priority: 17.03.2020 US 202062990783 P
(43) Date of publication of application: 25.01.2023
(73) Proprietor: Ohio State Innovation Foundation, Columbus, OH 43201 (US)
(72) Inventor: HIGUITA-CASTRO, Natalia, Columbus, Ohio 43204 (US); GALLEGO-PEREZ, Daniel, Columbus, Ohio 43204 (US)
(74) Representative: Moré, Solveig Helga
(86) International application number: PCT/US2021/022674
(87) International publication number: WO 2021/188616

(56) References cited:
- WO-A1-2018/094120
- WO-A1-2019/014486
- US-A1- 2016 137 716
- US-A1- 2019 175 731
- US-A1- 2019 269 739
- ORTEGA-PINEDA LILIBETH ET AL: "Designer Extracellular Vesicles Modulate Pro-Neuronal Cell Responses and Improve Intracranial Retention", vol. 11, no. 5, 1 March 2022 (2022-03-01), DE, pages 2100805, XP093056943, ISSN: 2192-2640, Retrieved from the Internet <URL:https://www.ncbi.nlm.nih.gov/pmc/articles/PMC9466406/pdf/nihms-1801213.pdf> DOI: 10.1002/adhm.202100805
- RICHARDSON JOSEPH J. ET AL: "Surface Engineering of Extracellular Vesicles through Chemical and Biological Strategies", CHEMISTRY OF MATERIALS, vol. 31, no. 7, 9 April 2019 (2019-04-09), US, pages 2191 - 2201, XP055817560, ISSN: 0897-4756, DOI: 10.1021/acs.chemmater.9b00050
- ISOLA ALLISON L. ET AL: "Exosomes released by metabotropic glutamate receptor 1 (GRM1) expressing melanoma cells increase cell migration and invasiveness", vol. 9, no. 1, 2 January 2018 (2018-01-02), pages 1187 - 1199, XP093057248, Retrieved from the Internet <URL:https://www.ncbi.nlm.nih.gov/pmc/articles/PMC5787429/pdf/oncotarget-09-1187.pdf> DOI: 10.18632/oncotarget.23455
- FAURE J ET AL: "Exosomes are released by cultured cortical neurones", MOLECULAR AND CELLULAR NEUROSCIENCES, SAN DIEGO, US, vol. 31, no. 4, 1 April 2006 (2006-04-01), pages 642 - 648, XP024908190, ISSN: 1044-7431, [retrieved on 20060401], DOI: 10.1016/J.MCN.2005.12.003
- ANONYMOUS: "Abstract 3889: GRM1 over-expression in melanoma cells promotes microvesicle formation which functions in stimulating angiogenesis. | Cancer Research | American Association for Cancer Research", 1 January 2013 (2013-01-01), XP093057528, Retrieved from the Internet <URL:https://aacrjournals.org/cancerres/article/73/8_Supplement/3889/589769/Abstract-3889-GRM1-over-expression-in-melanoma> [retrieved on 20230626]
- DATABASE BIOSIS [online] BIOSCIENCES INFORMATION SERVICE, PHILADELPHIA, PA, US; April 2013 (2013-04-01), KOO JASMINE J ET AL: "GRM1 over-expression in melanoma cells promotes microvesicle formation which functions in stimulating angiogenesis", Database accession no. PREV201400243977
- CANCER RESEARCH, vol. 73, no. 8, Suppl. 1, April 2013 (2013-04-01), 104TH ANNUAL MEETING OF THE AMERICAN-ASSOCIATION-FOR-CANCER-RESEARCH (AACR); WASHINGTON, DC, USA; APRIL 06 -10, 2013, pages 3889, ISSN: 0008-5472(print), DOI: 10.1158/1538-7445.AM2013-3889
- DATABASE MEDLINE [online] US NATIONAL LIBRARY OF MEDICINE (NLM), BETHESDA, MD, US; July 2013 (2013-07-01), FRÜHBEIS CARSTEN ET AL: "Neurotransmitter-triggered transfer of exosomes mediates oligodendrocyte-neuron communication.", Database accession no. NLM23874151
- FRÜHBEIS CARSTEN ET AL: "Neurotransmitter-triggered transfer of exosomes mediates oligodendrocyte-neuron communication.", PLOS BIOLOGY JUL 2013, vol. 11, no. 7, July 2013 (2013-07-01), pages e1001604, ISSN: 1545-7885
- DATABASE MEDLINE [online] US NATIONAL LIBRARY OF MEDICINE (NLM), BETHESDA, MD, US; 2 January 2018 (2018-01-02), ISOLA ALLISON L ET AL: "Exosomes released by metabotropic glutamate receptor 1 (GRM1) expressing melanoma cells increase cell migration and invasiveness.", Database accession no. NLM29416686
- ISOLA ALLISON L ET AL: "Exosomes released by metabotropic glutamate receptor 1 (GRM1) expressing melanoma cells increase cell migration and invasiveness.", ONCOTARGET 02 JAN 2018, vol. 9, no. 1, 2 January 2018 (2018-01-02), pages 1187 - 1199, XP093057248, ISSN: 1949-2553, DOI: 10.18632/oncotarget.23455
- DATABASE BIOSIS [online] BIOSCIENCES INFORMATION SERVICE, PHILADELPHIA, PA, US; July 2000 (2000-07-01), YATSUSHIRO SHOUKI ET AL: "Ionotropic glutamate receptors trigger microvesicle-mediated exocytosis of L-glutamate in rat pinealocytes", Database accession no. PREV200000324996
- YATSUSHIRO SHOUKI ET AL: "Ionotropic glutamate receptors trigger microvesicle-mediated exocytosis of L-glutamate in rat pinealocytes", JOURNAL OF NEUROCHEMISTRY, vol. 75, no. 1, July 2000 (2000-07-01), pages 288 - 297, ISSN: 0022-3042
- DATABASE BIOSIS [online] BIOSCIENCES INFORMATION SERVICE, PHILADELPHIA, PA, US; July 2013 (2013-07-01), FRUEHBEIS CARSTEN ET AL: "Neurotransmitter-Triggered Transfer of Exosomes Mediates Oligodendrocyte-Neuron Communication", Database accession no. PREV201300621667
- PLOS BIOLOGY, vol. 11, no. 7, July 2013 (2013-07-01), pages Article No.: e1001604, ISSN: 1545-7885(print), DOI: 10.1371/JOURNAL.PBIO.1001604
- DATABASE MEDLINE [online] US NATIONAL LIBRARY OF MEDICINE (NLM), BETHESDA, MD, US; 14 June 2019 (2019-06-14), MICCI MARIA-ADELAIDE ET AL: "Hippocampal stem cells promotes synaptic resistance to the dysfunctional impact of amyloid beta oligomers via secreted exosomes.", Database accession no. NLM31200742
- MICCI MARIA-ADELAIDE ET AL: "Hippocampal stem cells promotes synaptic resistance to the dysfunctional impact of amyloid beta oligomers via secreted exosomes.", MOLECULAR NEURODEGENERATION 14 06 2019, vol. 14, no. 1, 14 June 2019 (2019-06-14), pages 25, ISSN: 1750-1326
- DATABASE MEDLINE [online] US NATIONAL LIBRARY OF MEDICINE (NLM), BETHESDA, MD, US; 2021, BEARD KRYSHAWNA ET AL: "Extracellular vesicles as distinct biomarker reservoirs for mild traumatic brain injury diagnosis.", Database accession no. NLM34622206
- BEARD KRYSHAWNA ET AL: "Extracellular vesicles as distinct biomarker reservoirs for mild traumatic brain injury diagnosis.", BRAIN COMMUNICATIONS 2021, vol. 3, no. 3, 2021, pages fcab151, XP093057291, ISSN: 2632-1297, DOI: 10.1093/braincomms/fcab151

## Description

### BACKGROUND

Excitotoxicity is the pathological process by which nerve cells are damaged or killed by excessive stimulation by neurotransmitters such as glutamate and similar substances. This occurs when receptors for the excitatory neurotransmitter glutamate (glutamate receptors) such as the NMDA receptor and AMPA receptor are overactivated by glutamatergic storm. Excitotoxicity may be involved in spinal cord injury, stroke, traumatic brain injury, hearing loss (through noise overexposure or ototoxicity), and in neurodegenerative diseases of the central nervous system (CNS) such as multiple sclerosis, Alzheimer's disease, amyotrophic lateral sclerosis (ALS), Parkinson's disease, alcoholism or alcohol withdrawal and especially over-rapid benzodiazepine withdrawal, and also Huntington's disease.

### SUMMARY

The present invention is set out in the appended set of claims. In particular, disclosed herein is a composition comprising extracellular vesicles (EVs) produced from donor cells engineered to express a glutamate receptor (GluR), wherein the GluR is a metabotropic glutamate receptor-4 (GRM4) or a metabotropic glutamate receptor-8 (GRM8). The donor cells may be skin cells. In some embodiments, the EVs encapsulate a therapeutic or diagnostic cargo. In some embodiments, the therapeutic cargo comprises a proangiogenic, proneurogenic, or anti-inflammatory molecular cargo. In some embodiments, the diagnostic cargo comprises a molecular beacon. The composition of the present invention may be for use in the treatment of a CNS injury resulting in excitotoxicity in a subject, comprising administering to the subject an effective amount of the composition, wherein the EVs encapsulate a therapeutic cargo and wherein the CNS injury resulting in excitotoxicity is selected from the group consisting of spinal cord injury, stroke, traumatic brain injury and a neurodegenerative disease. In some embodiments, the neurodegenerative disease is any of Alzheimer's disease, Parkinson's disease, a Parkinson's-related disorder, Huntington's disease, prion disease, motor neuron disease (MND), spinocerebellar ataxia (SCA) or spinal muscular atrophy (SMA).

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1A and 1B illustrate nanoscale engineering of designer extracellular vesicles for therapeutic applications.

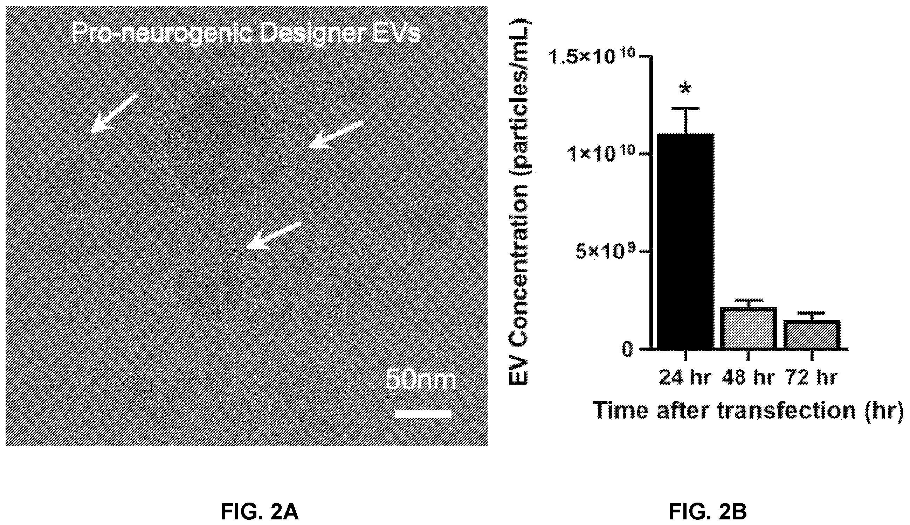

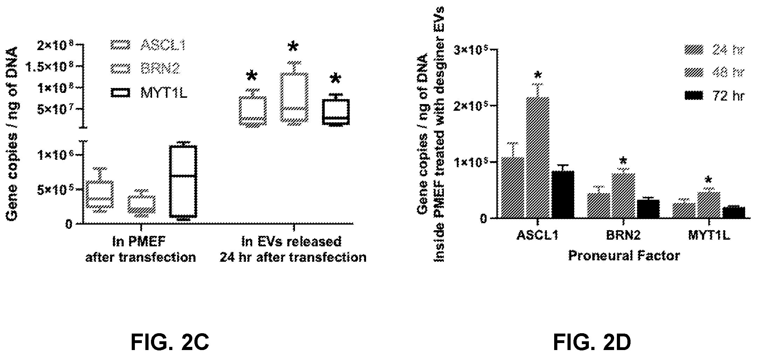

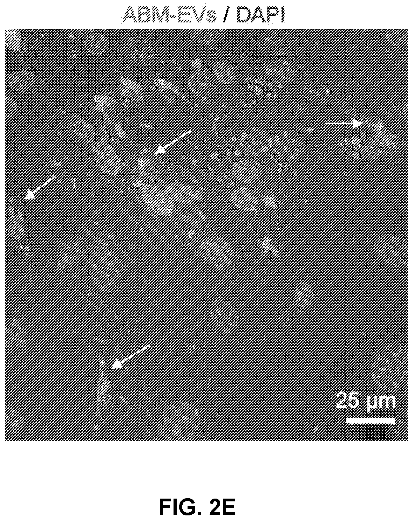

FIGs. 2A to 2E show proneural designer EVs characterization. FIG. 2A shows transmission electron micrograph of designer EVs derived from primary mouse embryonic fibroblasts (PMEFs) and loaded with the proneural factors the ASCL1, BRN2, and MYT1L (i.e., ABM cocktail). FIG. 2B shows dynamic of proneural designer EV release showing a peak 24 hours after nanotransfection of PMEF, with a particle concentration in the order of ten billion particles per mL (*p-value=0.018). FIG. 2C shows the number of gene copies packed inside these proneural designer EVs is approx. 3 orders of magnitude higher than that delivered to the donor cell for all three factors (*p-value=0.021). FIG. 2D shows proneural designer EVs are successfully captured by PMEFs in culture, with a peak in uptake 48 hours after treatment (*p-value ≤ 0.036). FIG. 2E Fluorescent image of PMEF cells incorporating fluorescently labeled (red) proneurogeninc-EVs 24 hours after treatment.

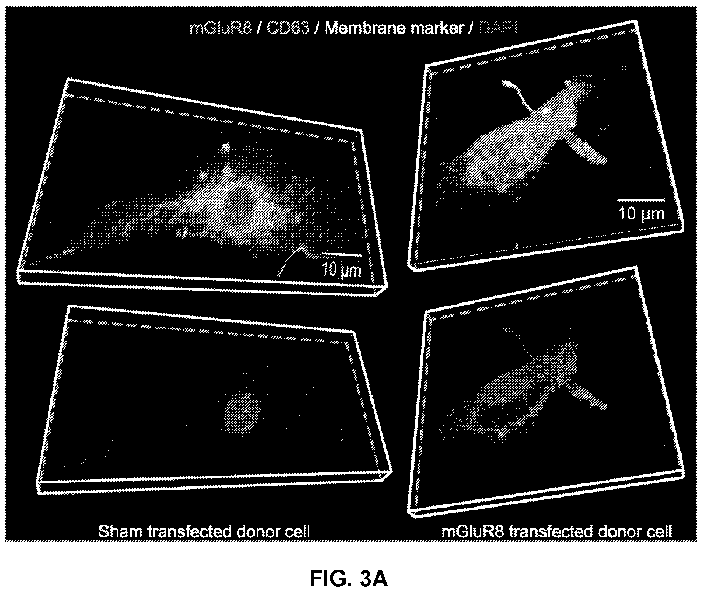

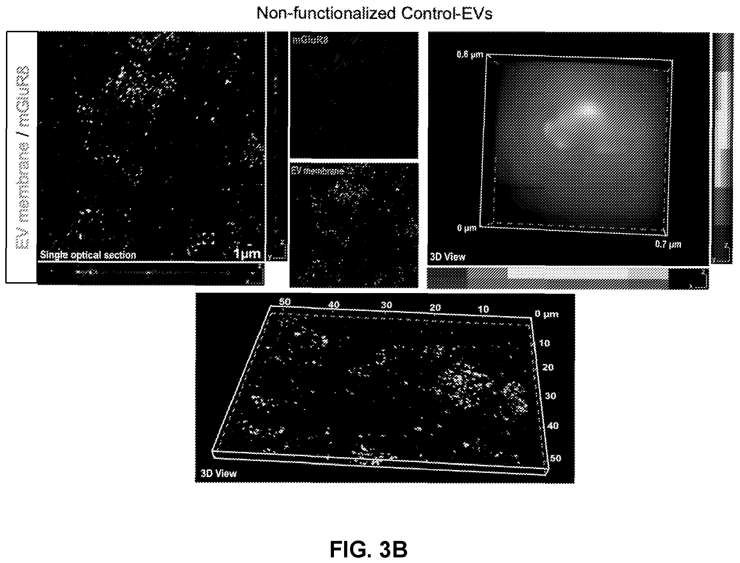

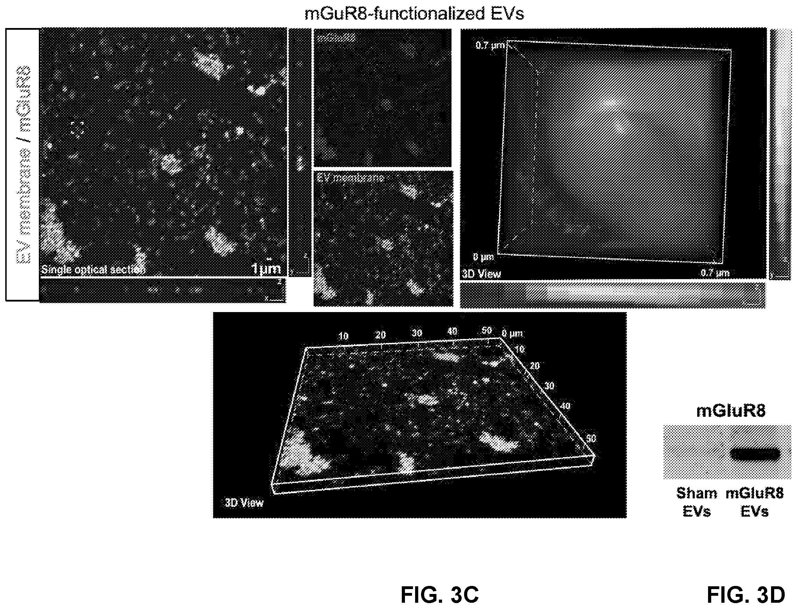

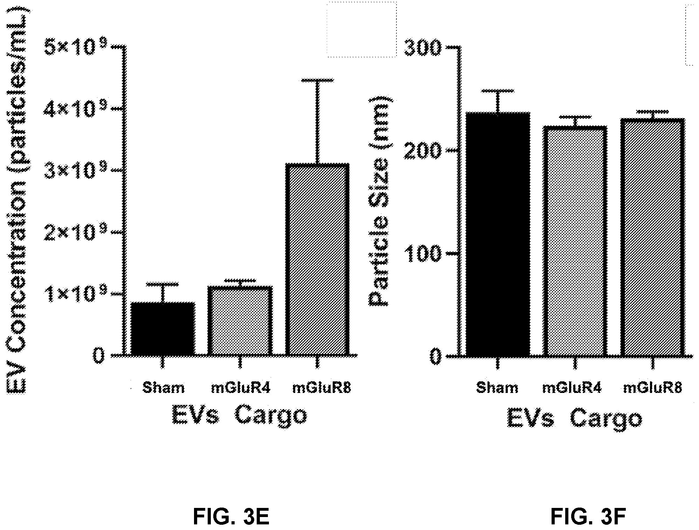

FIG. 3A shows confocal images of donor cells transfected with the mGluR8 or a sham vector, showing positive colocalization of cell membrane (green and white) and the transfected glutamate receptor (red) only for mGluR8 transfected cells. FIGs. 3B and 3C show Confocal images of EVs derived from sham or mGluR8 transfected donor cells, where EVs derived from GlutR8 transfected cells show co-localization of the EV membrane (green) with the targeting receptor (red). FIG. 3D Western blot of mGluR8-functionalized EVs showing positive protein expression compared to sham (control)-EVs. FIGs. 3E and 3F show characterization of designer EVs functionalized with mGRM4 and mGRM8 derived from PMEFs 24 hours after nanotransfection with plasmids encoding for each receptor or sham vector, with a particle concentration in the range of billions of EVs per mL and an average particle approximately 230 nm.

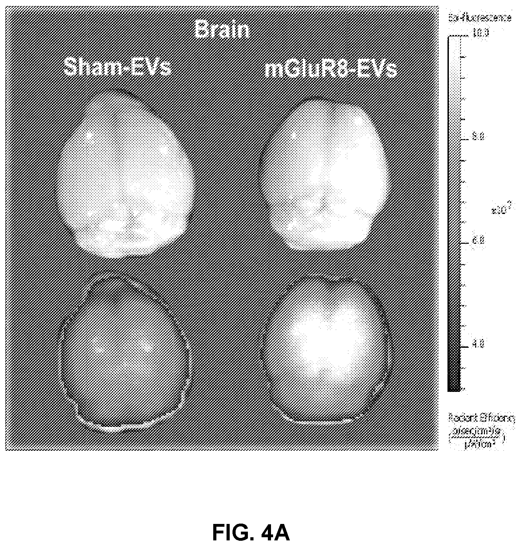

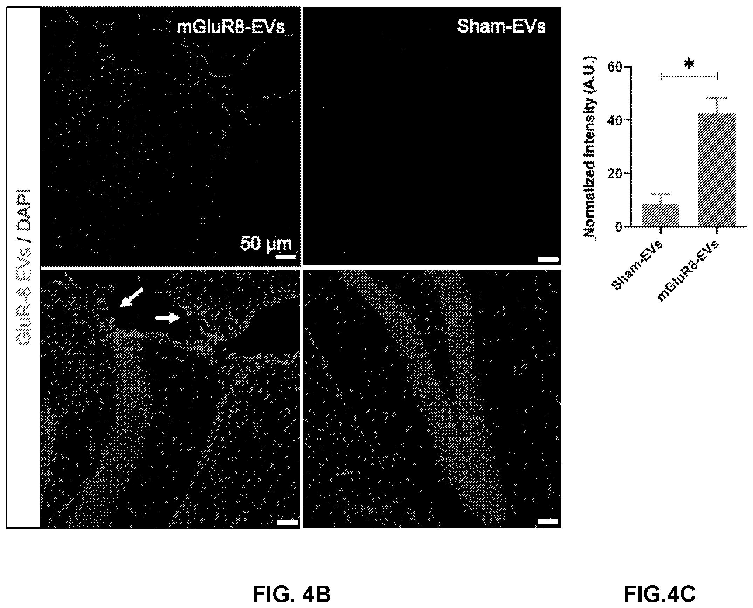

FIGs. 4A to 4C show functionalized designer EV to target the brain. FIG. 4A shows in vivo imaging of brains after intranasal delivery of fluorescently labeled designer EVs functionalized with mGluR8 or sham (control) EVs, showing significantly higher accumulation of functionalized EVs in the brain 24 hours after treatment. FIGs. 4B and 4C show immunofluorescence images of cerebellum and corpus callosum (sagittal cut) of brains of animals treated with fluorescently labeled (red) sham- or mGluR8-EVs, 24 hours post-intranasal instillation, and respective fluorescence intensity quantification (n=3, *p-value=0.0083).
FIG. 5 show characterization of relative expression of metabotropic glutamate receptors (mGluR4 and mGluR8) in designer EVs, these receptors are used to functionalize neurogenic designer EVs.
FIGs. 6A and 6B show designer EV biodistribution 24 hours after intranasal delivery showing higher accumulation in the brain of mice treated with designer EVs functionalized with mGluR8 vs. non-functionalized designer EVs, which accumulate in the liver tissue as they are cleared from the body.
FIGs. 7A and 7B show comparison of yield for in vitro-derived (using PMEF as donor cells) vs. in vivo-derived (using skin cells as donor cells) ABM- and control-designer EVs, showing that a significantly higher number of EVs are produced in vivo.
FIGs. 8A and 8B show efficiency of molecular loading of neurogenic factors ACL1, BRN2, and MYT1L (ABM) inside designer EVs vs. number of gene copies inside donor cells 24 hours after transfection.
FIG. 9A shows immunofluorescence images of primary neurons incorporating mGluR8 functionalized EVs (red) and control-EVs (green), showing preferential accumulation of GluR-8 EVs in postsynaptic regions (postsynaptic protein staining, PSD-95) (violet), with zoom-in regions for each type of sample (bottom) 7hours after treatment. FIG. 9B shows quantification of green (sham-EVs) or red (mGluR8-EVs) fluorescence intensity at the soma and neuronal projections (n=3, *p-value=0.024).
FIG. 10 shows mGluR4- and mGLuR8-functionalized designer EVs uptake by primary mouse embryonic neurons 8 hours after treatment.

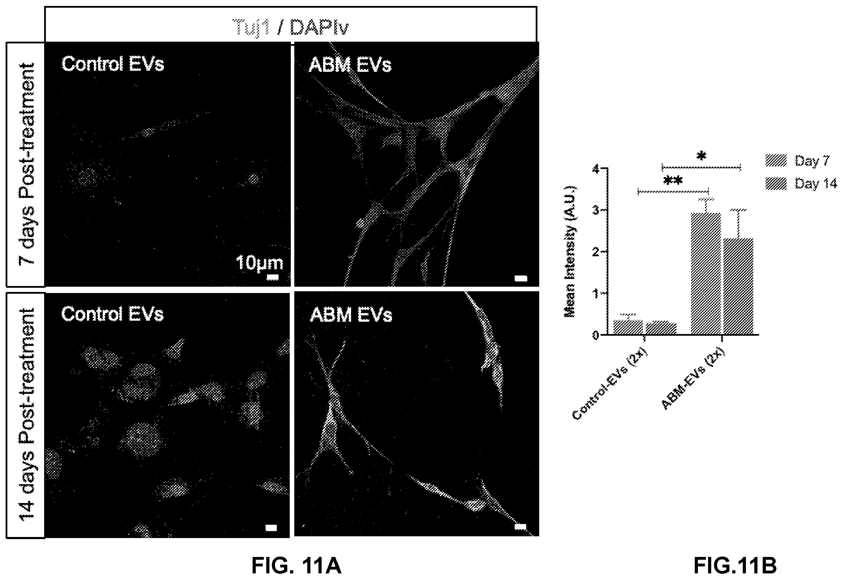

FIG. 11A shows how prolonged culture studies suggest that PMEFs exposed to ABM-loaded EVs exhibit pro-neuronal conversions, as evidence by the increased in immunoreactivity for Tuj1 (green), a neuronal marker, relative to PMEFs exposed to control EVs as early as 7 days after treatment. These data suggest that the extent of plasmid DNA transfer from EVs to recipient cells falls within the same order of magnitude compared to direct electroporation. Additionally, the induction of Tuj1 immunoreactivity in fibroblast cultures suggest that ABM-loaded EVs could potentially be used to drive pro-neuronal responses/conversions in non-neuronal cells. FIG. 11B shows the quantification of Tuj1 fluorescence intensity 7- and 14-days post-treatment (n=3, *p-value=0.043, **p-value=0.004).

### DETAILED DESCRIPTION

Before the present disclosure is described in greater detail, it is to be understood that the terminology used herein is for the purpose of describing particular embodiments only, and is not intended to be limiting, since the scope of the present disclosure will be limited only by the appended claims.

Unless defined otherwise, all technical and scientific terms used herein have the same meaning as commonly understood by one of ordinary skill in the art to which this disclosure belongs. Although any methods and materials similar or equivalent to those described herein can also be used in the practice or testing of the present disclosure, the preferred methods and materials are now described.

The citation of any publication is for its disclosure prior to the filing date and should not be construed as an admission that the present disclosure is not entitled to antedate such publication by virtue of prior disclosure. Further, the dates of publication provided could be different from the actual publication dates that may need to be independently confirmed.

As will be apparent to those of skill in the art upon reading this disclosure, each of the individual embodiments described and illustrated herein has discrete components and features which may be readily separated from or combined with the features of any of the other several embodiments.

Embodiments of the present disclosure will employ, unless otherwise indicated, techniques of chemistry, biology, and the like, which are within the skill of the art.

The following examples are put forth so as to provide those of ordinary skill in the art with a complete disclosure and description of how to perform the methods and use the probes disclosed and claimed herein. Efforts have been made to ensure accuracy with respect to numbers (e.g., amounts, temperature, etc.), but some errors and deviations should be accounted for. Unless indicated otherwise, parts are parts by weight, temperature is in °C, and pressure is at or near atmospheric. Standard temperature and pressure are defined as 20 °C and 1 atmosphere.

Before the embodiments of the present disclosure are described in detail, it is to be understood that, unless otherwise indicated, the present disclosure is not limited to particular materials, reagents, reaction materials, manufacturing processes, or the like, as such can vary. It is also to be understood that the terminology used herein is for purposes of describing particular embodiments only, and is not intended to be limiting. It is also possible in the present disclosure that steps can be executed in different sequence where this is logically possible.

It must be noted that, as used in the specification and the appended claims, the singular forms "a," "an," and "the" include plural referents unless the context clearly dictates otherwise.

The terms "extracellular vesicle" and "EV" are used herein to refer to a vesicle of about 10nm to 10µm in size consisting of fluid, macro-molecules, solutes,
and metabolites from a cell contained by a lipid bilayer or micelle. In some cases, the EV is a cell-derived EV. The term "EV" also includes lipid vesicle engineered to contain bioactive molecules found in a cell-derived EVs. These terms encompass both exosomes and ectosomes. Exosomes are released on the exocytosis of multivesicular bodies (MVBs). Ectosomes are vesicles assembled at and released from the plasma membrane. In some cases, the EV is about 20nm to 10µm, 20nm to 1µm, 20 nm-500 nm, 30 nm-100nm, 30 nm-160nm, or 80-160 nm in size. In some embodiments, the EVs are exosomes that are about 20 to 150 nm in size.

Furthermore, the following terms shall have the definitions set out below. It is understood that in the event a specific term is not defined herein below, that term shall have a meaning within its typical use within context by those of ordinary skill in the art.

The term "subject" refers to any individual who is the target of administration or treatment. The subject can be a vertebrate, for example, a mammal. Thus, the subject can be a human or veterinary patient. The term "patient" refers to a subject under the treatment of a clinician, e.g., physician.

The term "therapeutically effective" refers to the amount of the composition used is of sufficient quantity to ameliorate one or more causes or symptoms of a disease or disorder. Such amelioration only requires a reduction or alteration, not necessarily elimination.

The term "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problems or complications commensurate with a reasonable benefit/risk ratio.

The terms "treat", "treating", and "treatment", etc., as used herein, refer to any action providing a benefit to a patient at risk for or afflicted by a disease state, condition or deficiency which may be improved using cellular compositions according to the present invention. Treating a condition includes improving the condition through lessening or suppression of at least one symptom, delay in progression of the effects of the disease state or condition, including the prevention or delay in the onset of effects of the disease state or condition, etc. In the present application, treatment can involve reducing the impact of a spinal cord injury or stroke, including reversing and/or inhibiting the effects of such injury, reversing, improving, inhibiting and/or stabilizing a neurodegenerative disease such that the disease improves and/or does not progress or worsen. The term "prophylactic" is used to describe a method which "reduces the likelihood" that a particular result will occur, often the progression and/or worsening of a disease state and/or condition.

The term "autologous EV" is used to describe a population of EVs which are obtained from cells from a subject or patient to whom the EVs are to be administered.

### Extracellular Vesicles

Disclosed herein are designer extracellular vesicles (EVs) functionalized with GluRs, which can selectively target injured regions of the CNS experiencing excitotoxicity.

The production of functionalized EVs that may be used to deliver cargo to neurons has been previously described. For instance, Richardson and Ejima, 2019 teaches decorating the surface of EVs with targeting peptides and to use these functionalized EVs as delivery vehicles for therapeutic cargo during the treatment of diseases. The authors inter alia describe how exosomes can be targeted to neurons, microglia and oligodendrocytes by introducing a targeting peptide formed by the neuron-specific RVG peptide 102 fused to the N-terminus of the exosome membrane protein Lamp2b.32 on the surface of said exosomes (Richardson and Ejima, 2019, Surface Engineering of Extracellular Vesicles through Chemical and Biological Strategies. Chemistry of Materials 2019 31 (7), 2191-2201).

US 2016/137716 A1 discloses the use of exosomes as delivery or administration vehicles for biopharmaceuticals. The exosomes comprise attached to their membrane a polypeptide construct, wherein the polypeptide construct comprises at least one carrier polypeptide fused to at least one therapeutic polypeptide decoy receptor present at least partially on the outside of the exosome and wherein the at least one therapeutic polypeptide decoy receptor is signalling-incompetent. The exosomes disclosed in US 2016/137716 A1 are reported to be suitable for treating various diseases including, e.g., neurodegenerative diseases such as Alzheimer's disease, Parkinson's disease or Huntingtons disease.

Exosomes and microvesicles are EVs that differ based on their process of biogenesis and biophysical properties, including size and surface protein markers. Exosomes are homogenous small particles ranging from 40 to 150 nm in size and they are normally derived from the endocytic recycling pathway. In endocytosis, endocytic vesicles form at the plasma membrane and fuse to form early endosomes. These mature and become late endosomes where intraluminal vesicles bud off into an intra-vesicular lumen. Instead of fusing with the lysosome, these multivesicular bodies directly fuse with the plasma membrane and release exosomes into the extracellular space. Exosome biogenesis, protein cargo sorting, and release involve the endosomal sorting complex required for transport (ESCRT complex) and other associated proteins such as Alix and Tsg101. In contrast, microvesicles, are produced directly through the outward budding and fission of membrane vesicles from the plasma membrane, and hence, their surface markers are largely dependent on the composition of the membrane of origin. Further, they tend to constitute a larger and more heterogeneous population of extracellular vesicles, ranging from 150 to 1000 nm in diameter. However, both types of vesicles have been shown to deliver functional mRNA, miRNA and proteins to recipient cells.

The disclosed EVs can be obtained by culturing donor cells in cell culture medium under conditions and for a time sufficient to produce EVs, and isolating said EVs from the culture medium.

The donor cell may be autologous. However, the donor cell may also be allogeneic. For example, these could be cells isolated from tissue biopsies (e.g., skin) or other cells derived from specific organs from matching donors.

In some embodiments, the donor cells can be any cell able to produce EVs, including (but not limited to) skin cells (e.g., fibroblasts, keratinocytes, skin stem cells), adipocytes, dendritic cells, peripheral blood mononuclear cells (PBMC), pancreatic cells (e.g., ductal epithelial cells), liver cells (e.g., hepatocytes), immune cells (e.g., T cells, macrophages, myeloid derived suppressor cells), Endothelial cells, or intervertebral disc cells.

As described in U.S. Patent Application Document No. 20140356382, "[e]xosomes produced from cells can be collected from the culture medium and/or cell tissue by any suitable method. Typically a preparation of EVs can be prepared from cell culture or tissue supernatant by centrifugation, filtration or combinations of these methods. For example, EVs can be prepared by differential centrifugation, that is low speed (<2,0000 g) centrifugation to pellet larger particles followed by high speed (>100,000 g) centrifugation to pellet EVs, size filtration with appropriate filters (for example, 0.22 µm filter), gradient ultracentrifugation (for example, with sucrose gradient) or a combination of these methods." It is noted that the contents of EVs, i.e., EVs in which the lipid bilayer has been removed or eliminated and the contents obtained may also be used to engineer artificial EVs.

Further, as described in U.S. Patent Application Document No. 20140356382, exogenous protein and/or peptide and other cargo can be introduced into the EVs by a number of different techniques including electroporation or the use of a transfection reagent. Electroporation conditions may vary depending on the charge and size of the biotherapeutic cargo. Typical voltages are in the range of 20V/cm to 1,000V/cm, such as 20V/cm to 100V/cm with capacitance typically between 25 µF and 250 µF, such as between 25 µF and 125 µF. A voltage in the range of 150 mV to 250 mV, particularly a voltage of 200 mV is preferred for loading EVs with an antibody. Alternatively, the EVs may be loaded with exogenous protein and/or peptide using a transfection reagent. Despite the small size of the EVs, conventional transfection agents may be used for transfection of EVs with protein and/or peptide. EVs may also be loaded by transforming or transfecting a host cell with a nucleic acid construct which expresses therapeutic protein or peptide of interest, such that the therapeutic protein or peptide is taken up into the EVs as the EVs are produced from the cell.

The EV-producing cells disclosed herein may be cultured for about 1, 2, 3, 4, 5, 6, 7, 8, 9, 10, 11, 12, 13 or 14 days or for as long as about 1, 2, 3, 4, 5, 6, 7, 8 weeks or about 1, 2, 3, 4, 5, or 6 months, depending on the cell and its ability to produce EVs. The EV-producing cells may be cultured in suitable media and grown under conditions that are readily determined by one of ordinary skill in the art. Cell culture conditions may vary with cell type and the examples presented hereinafter illustrate suitable media and conditions.

EVs can be harvested at various time intervals (e.g. at about 2, 4, 6, 8 or 3, 6, 9, 12 day or longer intervals, depending upon the rate of production of EVs). Exemplary yields of EVs can range from at least about 1 ng EVs/1 million cells, at least about 10 ng EVs/1 million cells, at least about 50 ng EVs/1 million cells, at least about 100 ng EVs/1 million cells, at least about 500 ng EVs/1 million cells, at least about 750 ng EVs/1 million cells, at least about 800 ng EVs/1 million cells, at least about 900 ng EVs/1 million cells, at least about 1.0 µg EVs/1 million cells, at least about 1.5 µg EVs/1 million cells, at least about 2.0 µg EVs/1 million cells, at least about 2.5 µg EVs/1 million cells, at least e.g. about 3.0 µg EVs/1 million cells, at least about 5.0 µg EVs/1 million cells, and at least about 10.0 µg EVs/1 million cells, during a time period of about 24 hours to seven days of culture of proliferative and non-proliferative neural cells as otherwise described herein.

EVs may be harvested and collected by ultracentrifugation or differential centrifugation or any combination thereof, pelleted EVs are collected, and, optionally, collected pelleted EVs are washed with a suitable medium. For example, a preparation of EVs can be prepared from cell culture or tissue supernatant by centrifugation, filtration or combinations of these methods. The EVs can be prepared by differential centrifugation, that is low speed (<2,0000 g) centrifugation to pellet larger particles followed by high speed (>100,000 g) centrifugation to pellet EVs, size filtration with appropriate filters (for example, 0.22 µm filter), gradient ultracentrifugation (for example, with sucrose gradient) or a combination of these methods. EVs may be purified by differential centrifugation, micro and ultra-filtration, polymeric precipitation, microfluidic separation, immunocapture and size-exclusion chromatography. These and/or related methods for isolating and purifying EVs are described by Théry, et al., Current Protocols in Cell Biology, (2006) 3.221-3.22.29, copyright 2006 by John Wiley & Sons, Inc.; Sokolova, et al., Colloids and Surfaces B: Biointerfaces, 2011, 87, 146-150; Wiklander, et al., Journal of Extracellular Vesicles, 2015, 4, 26316, pp. 1-13; and Böing, et al., Journal of Extracellular Vesicles, 2014, 3, 23430, pp. 1-11. Other methods for isolation may be developed such as electrical field radiofrequency and acoustics.

### Glutamate Receptors

The disclosed designer extracellular vesicles (EVs) are functionalized with glutamate receptors (GluRs) that can selectively target injured regions of the CNS experiencing excitotoxicity. The GluR is a metabotropic glutamate receptor (mGluR), in particular a metabotropic glutamate receptor-4 (mGluR4) or a metabotropic glutamate receptor-8 (mGluR8).

Therefore, donor cells are modified with a transgene capable of expressing GluRs in the donor cell. For instance, a polynucleotide comprising a GluR gene operably linked to an expression control sequence is delivered to the donor cell. The polynucleotide may be in a DNA vector. DNA vectors, such as viral vectors and non-viral vectors are known in the art and can be used to produce donor cells capable of producing the disclosed designer EVs. The non-viral vector may be a recombinant bacterial plasmid. The non-viral vector may have a pCDNA3 backbone. The vector may comprise an internal ribosome entry site (IRES).

Examples of mouse and human nucleic acid and amino acid sequences for GluRs for use in the disclosed compositions are provided below.

The mouse metabotropic glutamate receptor 4 (mGluR4) may have an ORF nucleic acid sequence:

ATGTCCGGGAAGGGAGGCTGGGCCTGGTGGTGGGCCCGGCTGCCCCTCTGCC
TACTCCTCAGCCTTTATGGCTCCTGGGTGCCTTCATCCCTAGGAAAGCCCAAGGGTCA
CCCCCACATGAACTCTATCCGTATCGATGGAGACATCACCCTGGGAGGCCTGTTTCCC
GTCCACGGTCGCGGCTCCGAGGGCAAGGCCTGCGGCGAGTTGAAGAAGGAGAAAGGT

ATCCACCGGCTGGAGGCCATGCTCTTTGCCCTGGACCGCATCAACAACGACCCGGACC
TACTGCCCAACATCACGTTGGGCGCCCGCATTCTGGACACCTGCTCAAGGGATACCCA
CGCCCTGGAGCAGTCCCTGACCTTTGTGCAGGCGCTCATCGAGAAGGACGGCACGGA
GGTCCGCTGCGGCAGCGGGGGCCCACCCATCATCACCAAGCCTGAACGAGTGGTGGG
TGTCATCGGAGCTTCGGGGAGCTCCGTCTCGATCATGGTGGCCAACATCCTCCGCCTC
TTCAAGATCCCCCAGATCAGCTACGCCTCCACGGCTCCCGACTTGAGTGATAACAGCC
GCTATGACTTCTTCTCCCGGGTCGTGCCCTCGGACACATACCAGGCCCAGGCCATGGT
GGACATCGTCCGGGCCCTCAAGTGGAACTATGTGTCCACGCTGGCCTCAGAGGGTAG
CTACGGCGAGAGCGGCGTGGAGGCCTTTATCCAGAAGTCCCGAGAGAACGGAGGCGT
GTGCATTGCCCAGTCGGTGAAGATTCCACGGGAACCCAAGACCGGGGAGTTTGACAAG
ATCATCAAACGCCTTCTGGAAACGTCCAATGCCAGAGCCATCATCATCTTTGCCAACGA
GGATGATATCAGGAGGGTGCTGGAGGCAGCGCGCAGGGCCAACCAGACCGGCCACTT
CTTTTGGATGGGTTCTGATAGCTGGGGCTCCAAGAGCGCCCCCGTGCTGCGCCTTGAG
GAAGTGGCTGAAGGTGCAGTCACCATTCTTCCCAAGAGGACGTCTGTGCGAGGGTTTG
ACCGATACTTCTCCAGCCGCACGCTTGACAACAACAGGCGCAACATCTGGTTTGCTGA
GTTCTGGGAGGACAACTTCCATTGCAAGTTGAGCCGCCACGCGCTCAAGAAGGGAAGC
CACATCAAGAAGTGCACCAACCGAGAGCGCATCGGGCAGGACTCGGCCTACGAACAG
GAGGGGAAGGTGCAGTTTGTGATCGACGCCGTGTACGCCATGGGCCATGCTCTGCAC
GCCATGCATCGTGACCTGTGTCCCGGCCGCGTAGGACTCTGCCCTCGAATGGACCCT
GTGGATGGCACCCAGCTGCTTAAGTACATCAGAAACGTCAACTTCTCAGGCATCGCCG
GGAACCCGGTGACCTTCAACGAGAACGGAGACGCGCCAGGGCGTTATGACATCTACC
AGTACCAACGTCGCAACGGCTCGGCTGAGTACAAGGTCATCGGCTCATGGACAGACCA
CTTGCACCTCAGAATAGAGCGGATGCAGTGGCCAGGGAGTGGCCAGCAGCTGCCACG
CTCCATCTGCAGCCTGCCCTGCCAGCCAGGCGAGCGGAAGAAGACGGTGAAGGGCAT
GGCTTGCTGCTGGCACTGCGAGCCCTGCACGGGGTACCAGTACCAGGTGGACCGCTA
CACCTGTAAGACCTGCCCCTATGACATGCGGCCCACGGAGAACCGCACGAGCTGCCA
GCCCATACCCATTGTCAAGTTGGAGTGGGACTCACCCTGGGCTGTGCTGCCCCTCTTC
CTGGCTGTGGTGGGCATTGCTGCCACGCTGTTCGTGGTGGTCACTTTTGTGCGCTACA
ACGACACTCCGATCGTCAAGGCCTCGGGCCGGGAGCTGAGCTACGTCCTGCTGGCGG
GCATCTTTCTCTGCTATGCCACCACCTTCCTCATGATCGCAGAGCCTGACCTGGGGAC
CTGTTCACTCCGCCGCATCTTCCTGGGGCTTGGCATGAGCATCAGCTACGCGGCCCTG
CTGACCAAGACCAACCGCATCTACCGCATCTTTGAGCAGGGCAAGCGGTCGGTCAGCG
CCCCACGGTTCATCAGCCCCGCCTCGCAGCTGGCCATCACCTTCGTCCTCATCTCGCT
GCAGTTGCTTGGCATCTGCGTGTGGTTCGTGGTGGACCCCTCCCACTCGGTGGTGGAC
TTCCAGGACCAGCGGACACTTGACCCCCGCTTCGCCCGGGGTGTGCTCAAGTGTGAC

ATCTCGGACCTGTCGCTCATCTGCCTCCTGGGCTACAGCATGCTGCTGATGGTCACGT
GTACTGTGTATGCCATCAAGACTCGAGGCGTGCCTGAGACCTTCAATGAGGCCAAGCC
CATCGGCTTCACCATGTACACCACCTGCATCGTCTGGCTGGCCTTCATCCCCATCTTTT
TTGGCACCTCGCAGTCGGCTGACAAGGTAACCTCTGAGGCCCTGCCCGTGGAATTCAG
CCCGCCATTGCTGGCACATAAT (SEQ ID NO:1).

The mouse metabotropic glutamate receptor 4 (mGluR4) may have an amino acid sequence:

MSGKGGWAWWWARLPLCLLLSLYGSWVPSSLGKPKGHPHMNSIRIDGDITLGGLFPVHG
RGSEGKACGELKKEKGIHRLEAMLFALDRINNDPDLLPNITLGARILDTCSRDTHALEQSLTF
VQALIEKDGTEVRCGSGGPPIITKPERVVGVIGASGSSVSIMVANILRLFKIPQISYASTAPDL
SDNSRYDFFSRVVPSDTYQAQAMVDIVRALKWNYVSTLASEGSYGESGVEAFIQKSRENG
GVCIAQSVKIPREPKTGEFDKIIKRLLETSNARAIIIFANEDDIRRVLEAARRANQTGHFFWMG
SDSWGSKSAPVLRLEEVAEGAVTILPKRTSVRGFDRYFSSRTLDNNRRNIWFAEFWEDNF
HCKLSRHALKKGSHIKKCTNRERIGQDSAYEQEGKVQFVIDAVYAMGHALHAMHRDLCPG
RVGLCPRMDPVDGTQLLKYIRNVNFSGIAGNPVTFNENGDAPGRYDIYQYQRRNGSAEYK
VIGSWTDHLHLRIERMQWPGSGQQLPRSICSLPCQPGERKKTVKGMACCWHCEPCTGYQ
YQVDRYTCKTCPYDMRPTENRTSCQPIPIVKLEWDSPWAVLPLFLAVVGIAATLFVVVTFVR
YNDTPIVKASGRELSYVLLAGIFLCYATTFLMIAEPDLGTCSLRRIFLGLGMSISYAALLTKTN
RIYRIFEQGKRSVSAPRFISPASQLAITFVLISLQLLGICVWFVVDPSHSVVDFQDQRTLDPRF
ARGVLKCDISDLSLICLLGYSMLLMVTCTVYAIKTRGVPETFNEAKPIGFTMYTTCIVWLAFIPI
FFGTSQSADKVTSEALPVEFSPPLLAHN (SEQ ID NO:2).

The mouse metabotropic glutamate receptor 8 (mGluR8) may have an ORF nucleic acid sequence:

ATGGTTTGTGAGGGAAAGCGCTCAACCTCTTGCCCTTGTTTCTTCCTTTTGACTGCCAA
GTTCTACTGGATCCTCACAATGATGCAAAGAACTCACAGCCAGGAGTATGCGCATTCCA
TCCGCCTGGATGGGGACATCATTTGGGGGTCTTTTTCCTGTTCATGCCAAGGGAGA
AAGAGGGGTGCCTTGTGGGGACCTGAAGAAGGAAAAGGGCATCCACAGACTTGAGGC
CATGCTTTATGCAATCGACCAGATTAATAAGGACCCCGATCTCCTCTCCAATATCACTCT
GGGTGTCCGGATCCTTGACACATGTTCCAGGGACACCTATGCTTTGGAGCAGTCACTA
ACCTTCGTGCAGGCACTGATAGAGAAAGACGCGTCTGACGTGAAGTGTGCTAATGGAG
ACCCACCCATATTCACCAAGCCCGACAAGATTTCTGGTGTCATAGGTGCTGCAGCAAG
CTCCGTGTCCATCATGGTGGCTAACATTTTAAGACTTTTTAAGATACCTCAGATTAGCTA
TGCATCTACAGCCCCAGAGCTAAGTGACAACACCAGGTATGATTTCTTTTCTCGGGTGG
TCCCGCCTGACTCCTACCAAGCCCAAGCCATGGTGGACATTGTGACAGCCCTGGGATG
GAATTATGTGTCAACACTGGCTTCCGAGGGGAACTATGGAGAGAGTGGTGTTGAGGCC

TTCACTCAGATCTCAAGGGAGATTGGTGGTGTTTGCATTGCTCAATCACAGAAAATCCC
ACGTGAACCAAGACCTGGAGAATTCGAAAAAATTATCAAACGCCTGCTGGAGACACCCA
ACGCTCGCGCAGTGATTATGTTTGCCAATGAGGATGACATCAGGAGGATATTGGAAGC
AGCAAAAAAATTAAACCAGAGTGGGCATTTTCTATGGATTGGCTCAGATAGTTGGGGAT
CCAAAATAGCACCTGTCTATCAGCAGGAGGAGATCGCCGAAGGAGCTGTGACAATTTT
GCCCAAAAGAGCATCAATTGATGGGTTTGACCGATACTTTAGAAGCCGAACTCTTGCCA
ATAATCGAAGAAATGTGTGGTTTGCAGAATTTTGGGAGGAGAATTTTGGATGCAAATTA
GGATCACATGGGAAGAGGAACAGTCATATAAAGAAATGCACAGGGCTGGAGCGAATTG
CACGGGATTCATCTTACGAACAAGAAGGAAAGGTTCAATTTGTAATTGATGCAGTGTATT
CCATGGCTTATGCACTGCACAACATGCACAAAGAACTCTGCCCTGGTTACATAGGCCTT
TGCCCAAGGATGGTTACCATCGATGGGAAAGAGCTACTGGGTTACATCAGGGCCGTGA
ATTTTAATGGCAGCGCTGGTACACCTGTCACTTTTAATGAGAATGGAGATGCTCCGGGA
CGCTACGATATCTTCCAATATCAGATAAACAACAAAAGTACAGAATACAAAATCATCGGC
CACTGGACCAATCAACTTCACCTAAAAGTGGAAGACATGCAGTGGGCTAATAGAGAGC
ACACGCACCCAGCATCTGTCTGCAGCCTGCCGTGCAAGCCTGGGGAGAGGAAGAAAA
CCGTGAAAGGGGTCCCTTGCTGCTGGCACTGTGAACGCTGCGAGGGTTATAACTACCA
GGTGGACGAACTCTCCTGTGAACTCTGCCCTTTGGATCAGAGACCAAACATCAACCGC
ACTGGCTGCCAGAGGATTCCCATCATCAAGTTGGAGTGGCATTCACCCTGGGCCGTGG
TACCTGTGTTCATAGCAATATTGGGAATCATTGCCACCACCTTTGTGATTGTGACCTTTG
TCCGCTATAATGACACACCAATCGTGAGAGCTTCTGGGCGGGAACTTAGTTATGTGCTC
CTAACGGGGATTTTTCTCTGTTACTCAATCACTTTTTTGATGATTGCGGCACCTGACACA
ATCATCTGCTCTTTCCGAAGGATCTTCCTGGGACTTGGTATGTGTTTCAGCTATGCAGC
ACTTTTGACCAAAACAAACCGTATCCACCGAATATTCGAGCAAGGGAAGAAATCTGTCA
CAGCACCTAAGTTCATCAGCCCAGCATCCCAGCTGGTGATCACCTTCAGCCTCATCTCC
GTACAGCTCCTTGGAGTGTTTGTGTGGTTTGTCGTGGATCCCCCCCACACCATCATTGA
CTATGGAGAACAGCGAACACTGGATCCCGAGAACGCCAGGGGAGTGCTCAAGTGTGA
CATTTCCGATCTGTCACTCATTTGTTCACTGGGATACAGTATCCTCCTGATGGTCACTTG
TACTGTTTATGCCATTAAAACCAGAGGGGTTCCAGAAACGTTCAATGAAGCCAAACCTA
TTGGATTTACCATGTACACCACGTGCATCATTTGGTTAGCTTTCATTCCCATCTTTTTTG
GTACAGCCCAGTCAGCAGAAAAGATGTACATCCAGACAACAACACTTACTGTCTCCATG
AGTTTAAGTGCTTCAGTGTCTCTGGGAATGCTCTATATGCCCAAAGTTTATATTATAATTT
TTCATCCAGAGCAGAACGTTCAAAAACGCAAGAGAAGCTTCAAGGCTGTGGTCACGGC
CGCTACCATGCAAAGCAAACTGATCCAAAAGGGAAATGACAGACCAAACGGCGAGGTG
AAAAGTGAACTCTGTGAGAGTCTTGAAACCAACAGTAAGTCATCTGTAGACTTTCAGAT
GGTCAAGAGCGGGAGCACTTCC (SEQ ID NO:3).

The mouse metabotropic glutamate receptor 8 (mGluR8) may have an amino acid sequence:

MVCEGKRSTSCPCFFLLTAKFYWILTMMQRTHSQEYAHSIRLDGDIILGGLFPVHAKGERGV
PCGDLKKEKGIHRLEAMLYAIDQINKDPDLLSNITLGVRILDTCSRDTYALEQSLTFVQALIEK
DASDVKCANGDPPIFTKPDKISGVIGAAASSVSIMVANILRLFKIPQISYASTAPELSDNTRYD
FFSRVVPPDSYQAQAMVDIVTALGWNYVSTLASEGNYGESGVEAFTQISREIGGVCIAQSQ
KIPREPRPGEFEKIIKRLLETPNARAVIMFANEDDIRRILEAAKKLNQSGHFLWIGSDSWGSKI
APVYQQEEIAEGAVTILPKRASIDGFDRYFRSRTLANNRRNVWFAEFWEENFGCKLGSHGK
RNSHIKKCTGLERIARDSSYEQEGKVQFVIDAVYSMAYALHNMHKELCPGYIGLCPRMVTID
GKELLGYIRAVNFNGSAGTPVTFNENGDAPGRYDIFQYQINNKSTEYKIIGHWTNQLHLKVE
DMQWANREHTHPASVCSLPCKPGERKKTVKGVPCCWHCERCEGYNYQVDELSCELCPL
DQRPNINRTGCQRIPIIKLEWHSPWAVVPVFIAILGIIATTFVIVTFVRYNDTPIVRASGRELSY
VLLTGIFLCYSITFLMIAAPDTIICSFRRIFLGLGMCFSYAALLTKTNRIHRIFEQGKKSVTAPKFI
SPASQLVITFSLISVQLLGVFVWFVVDPPHTIIDYGEQRTLDPENARGVLKCDISDLSLICSLG
YSILLMVTCTVYAIKTRGVPETFNEAKPIGFTMYTTCIIWLAFIPIFFGTAQSAEKMYIQTTTLT
VSMSLSASVSLGMLYMPKVYIIIFHPEQNVQKRKRSFKAVVTAATMQSKLIQKGNDRPNGEV
KSELCESLETNSKSSVDFQMVKSGSTS (SEQ ID NO:4).

The human metabotropic glutamate receptor 4 (mGluR4) may have an ORF nucleic acid sequence:

ATGCCTGGGAAGAGAGGCTTGGGCTGGTGGTGGGCCCGGCTGCCCCTTTGCCTGCTC
CTCAGCCTTTACGGCCCCTGGATGCCTTCCTCCCTGGGAAAGCCCAAAGGCCACCCTC
ACATGAATTCCATCCGCATAGATGGGGACATCACACTGGGAGGCCTGTTCCCGGTGCA
TGGCCGGGGCTCAGAGGGCAAGCCCTGTGGAGAACTTAAGAAGGAAAAGGGCATCCA
CCGGCTGGAGGCCATGCTGTTCGCCCTGGATCGCATCAACAACGACCCGGACCTGCT
GCCTAACATCACGCTGGGCGCCCGCATTCTGGACACCTGCTCCAGGGACACCCATGC
CCTCGAGCAGTCGCTGACCTTTGTGCAGGCGCTCATCGAGAAGGATGGCACAGAGGT
CCGCTGTGGCAGTGGCGGCCCACCCATCATCACCAAGCCTGAACGTGTGGTGGGTGT
CATCGGTGCTTCAGGGAGCTCGGTCTCCATCATGGTGGCCAACATCCTTCGCCTCTTC
AAGATACCCCAGATCAGCTACGCCTCCACAGCGCCAGACCTGAGTGACAACAGCCGCT
ACGATTTCTTCTCCCGCGTGGTGCCCTCGGACACGTACCAGGCCCAGGCCATGGTGGA
CATCGTCCGTGCCCTCAAGTGGAACTATGTGTCCACAGTGGCCTCGGAGGGCAGCTAT
GGTGAGAGCGGTGTGGAGGCCTTCATCCAGAAGTCCCGTGAGGACGGGGGCGTGTGC
ATCGCCCAGTCGGTGAAGATACCACGGGAGCCCAAGGCAGGCGAGTTCGACAAGATC
ATCCGCCGCCTCCTGGAGACTTCGAACGCCAGGGCAGTCATCATCTTTGCCAACGAGG
ATGACATCAGGCGTGTGCTGGAGGCAGCACGAAGGGCCAACCAGACAGGCCATTTCTT

CTGGATGGGCTCTGACAGCTGGGGCTCCAAGATTGCACCTGTGCTGCACCTGGAGGA
GGTGGCTGAGGGTGCTGTCACGATCCTCCCCAAGAGGATGTCCGTACGAGGCTTCGA
CCGCTACTTCTCCAGCCGCACGCTGGACAACAACCGGCGCAACATCTGGTTTGCCGAG
TTCTGGGAGGACAACTTCCACTGCAAGCTGAGCCGCCACGCCCTCAAGAAGGGCAGC
CACGTCAAGAAGTGCACCAACCGTGAGCGAATTGGGCAGGATTCAGCTTATGAGCAGG
AGGGGAAGGTGCAGTTTGTGATCGATGCCGTGTACGCCATGGGCCACGCGCTGCACG
CCATGCACCGTGACCTGTGTCCCGGCCGCGTGGGGCTCTGCCCGCGCATGGACCCTG
TAGATGGCACCCAGCTGCTTAAGTACATCCGAAACGTCAACTTCTCAGGCATCGCAGG
GAACCCTGTGACCTTCAATGAGAATGGAGATGCGCCTGGGCGCTATGACATCTACCAA
TACCAGCTGCGCAACGATTCTGCCGAGTACAAGGTCATTGGCTCCTGGACTGACCACC
TGCACCTTAGAATAGAGCGGATGCACTGGCCGGGGAGCGGGCAGCAGCTGCCCCGCT
CCATCTGCAGCCTGCCCTGCCAACCGGGTGAGCGGAAGAAGACAGTGAAGGGCATGC
CTTGCTGCTGGCACTGCGAGCCTTGCACAGGGTACCAGTACCAGGTGGACCGCTACAC
CTGTAAGACGTGTCCCTATGACATGCGGCCCACAGAGAACCGCACGGGCTGCCGGCC
CATCCCCATCATCAAGCTTGAGTGGGGCTCGCCCTGGGCCGTGCTGCCCCTCTTCCTG
GCCGTGGTGGGCATCGCTGCCACGTTGTTCGTGGTGATCACCTTTGTGCGCTACAACG
ACACGCCCATCGTCAAGGCCTCGGGCCGTGAACTGAGCTACGTGCTGCTGGCAGGCA
TCTTCCTGTGCTATGCCACCACCTTCCTCATGATCGCTGAGCCCGACCTTGGCACCTGC
TCGCTGCGCCGAATCTTCCTGGGACTAGGGATGAGCATCAGCTATGCAGCCCTGCTCA
CCAAGACCAACCGCATCTACCGCATCTTCGAGCAGGGCAAGCGCTCGGTCAGTGCCC
CACGCTTCATCAGCCCCGCCTCACAGCTGGCCATCACCTTCAGCCTCATCTCGCTGCA
GCTGCTGGGCATCTGTGTGTGGTTTGTGGTGGACCCCTCCCACTCGGTGGTGGACTTC
CAGGACCAGCGGACACTCGACCCCCGCTTCGCCAGGGGTGTGCTCAAGTGTGACATC
TCGGACCTGTCGCTCATCTGCCTGCTGGGCTACAGCATGCTGCTCATGGTCACGTGCA
CCGTGTATGCCATCAAGACACGCGGCGTGCCCGAGACCTTCAATGAGGCCAAGCCCAT
TGGCTTCACCATGTACACCACTTGCATCGTCTGGCTGGCCTTCATCCCCATCTTCTTTG
GCACCTCGCAGTCGGCCGACAAGCTGTACATCCAGACGACGACGCTGACGGTCTCGG
TGAGTCTGAGCGCCTCGGTGTCCCTGGGAATGCTCTACATGCCCAAAGTCTACATCAT
CCTCTTCCACCCGGAGCAGAATGTGCCCAAGCGCAAGCGCAGCCTCAAAGCCGTCGTT
ACGGCGGCCACCATGTCCAACAAGTTCACGCAGAAGGGCAACTTCCGGCCCAACGGA
GAGGCCAAGTCTGAGCTCTGCGAGAACCTTGAGGCCCCAGCGCTGGCCACCAAACAG
ACTTACGTCACTTACACCAACCATGCAATC (SEQ ID NO:5).

The human metabotropic glutamate receptor 4 (mGluR4) may have an amino acid sequence:

MPGKRGLGWWWARLPLCLLLSLYGPWMPSSLGKPKGHPHMNSIRIDGDITLGGLFPVHGR
GSEGKPCGELKKEKGIHRLEAMLFALDRINNDPDLLPNITLGARILDTCSRDTHALEQSLTFV
QALIEKDGTEVRCGSGGPPIITKPERVVGVIGASGSSVSIMVANILRLFKIPQISYASTAPDLS
DNSRYDFFSRVVPSDTYQAQAMVDIVRALKWNYVSTVASEGSYGESGVEAFIQKSREDGG
VCIAQSVKIPREPKAGEFDKIIRRLLETSNARAVIIFANEDDIRRVLEAARRANQTGHFFWMG
SDSWGSKIAPVLHLEEVAEGAVTILPKRMSVRGFDRYFSSRTLDNNRRNIWFAEFWEDNFH
CKLSRHALKKGSHVKKCTNRERIGQDSAYEQEGKVQFVIDAVYAMGHALHAMHRDLCPGR
VGLCPRMDPVDGTQLLKYIRNVNFSGIAGNPVTFNENGDAPGRYDIYQYQLRNDSAEYKVI
GSWTDHLHLRIERMHWPGSGQQLPRSICSLPCQPGERKKTVKGMPCCWHCEPCTGYQY
QVDRYTCKTCPYDMRPTENRTGCRPIPIIKLEWGSPWAVLPLFLAVVGIAATLFVVITFVRYN
DTPIVKASGRELSYVLLAGIFLCYATTFLMIAEPDLGTCSLRRIFLGLGMSISYAALLTKTNRIY
RIFEQGKRSVSAPRFISPASQLAITFSLISLQLLGICVWFVVDPSHSVVDFQDQRTLDPRFAR
GVLKCDISDLSLICLLGYSMLLMVTCTVYAIKTRGVPETFNEAKPIGFTMYTTCIVWLAFIPIFF
GTSQSADKLYIQTTTLTVSVSLSASVSLGMLYMPKVYIILFHPEQNVPKRKRSLKAVVTAATM
SNKFTQKGNFRPNGEAKSELCENLEAPALATKQTYVTYTNHAI (SEQ ID NO:6).

The human metabotropic glutamate receptor 8 (mGluR8) may have an ORF nucleic acid sequence:

ATGGTATGCGAGGGAAAGCGATCAGCCTCTTGCCCTTGTTTCTTCCTCTTGACCGCCAA
GTTCTACTGGATCCTCACAATGATGCAAAGAACTCACAGCCAGGAGTATGCCCATTCCA
TACGGGTGGATGGGGACATTATTTTGGGGGGTCTCTTCCCTGTCCACGCAAAGGGAGA
GAGAGGGGTGCCTTGTGGGGAGCTGAAGAAGGAAAAGGGGATTCACAGACTGGAGGC
CATGCTTTATGCAATTGACCAGATTAACAAGGACCCTGATCTCCTTTCCAACATCACTCT
GGGTGTCCGCATCCTCGACACGTGCTCTAGGGACACCTATGCTTTGGAGCAGTCTCTA
ACATTCGTGCAGGCATTAATAGAGAAAGATGCTTCGGATGTGAAGTGTGCTAATGGAGA
TCCACCCATTTTCACCAAGCCCGACAAGATTTCTGGCGTCATAGGTGCTGCAGCAAGCT
CCGTGTCCATCATGGTTGCTAACATTTTAAGACTTTTTAAGATACCTCAAATCAGCTATG
CATCCACAGCCCCAGAGCTAAGTGATAACACCAGGTATGACTTTTTCTCTCGAGTGGTT
CCGCCTGACTCCTACCAAGCCCAAGCCATGGTGGACATCGTGACAGCACTGGGATGGA
ATTATGTTTCGACACTGGCTTCTGAGGGGAACTATGGTGAGAGCGGTGTGGAGGCCTT
CACCCAGATCTCGAGGGAGATTGGTGGTGTTTGCATTGCTCAGTCACAGAAAATCCCA
CGTGAACCAAGACCTGGAGAATTTGAAAAAATTATCAAACGCCTGCTAGAAACACCTAA
TGCTCGAGCAGTGATTATGTTTGCCAATGAGGATGACATCAGGAGGATATTGGAAGCA
GCAAAAAAACTAAACCAAAGTGGGCATTTTCTCTGGATTGGCTCAGATAGTTGGGGATC
CAAAATAGCACCTGTCTATCAGCAAGAGGAGATTGCAGAAGGGGCTGTGACAATTTTGC
CCAAACGAGCATCAATTGATGGATTTGATCGATACTTTAGAAGCCGAACTCTTGCCAAT

AATCGAAGAAATGTGTGGTTTGCAGAATTCTGGGAGGAGAATTTTGGCTGCAAGTTAGG
ATCACATGGGAAAAGGAACAGTCATATAAAGAAATGCACAGGGCTGGAGCGAATTGCT
CGGGATTCATCTTATGAACAGGAAGGAAAGGTCCAATTTGTAATTGATGCTGTATATTCC
ATGGCTTACGCCCTGCACAATATGCACAAAGATCTCTGCCCTGGATACATTGGCCTTTG
TCCACGAATGAGTACCATTGATGGGAAAGAGCTACTTGGTTATATTCGGGCTGTAAATT
TTAATGGCAGTGCTGGCACTCCTGTCACTTTTAATGAAAACGGAGATGCTCCTGGACGT
TATGATATCTTCCAGTATCAAATAACCAACAAAAGCACAGAGTACAAAGTCATCGGCCA
CTGGACCAATCAGCTTCATCTAAAAGTGGAAGACATGCAGTGGGCTCATAGAGAACATA
CTCACCCGGCGTCTGTCTGCAGCCTGCCGTGTAAGCCAGGGGAGAGGAAGAAAACGG
TGAAAGGGGTCCCTTGCTGCTGGCACTGTGAACGCTGTGAAGGTTACAACTACCAGGT
GGATGAGCTGTCCTGTGAACTTTGCCCTCTGGATCAGAGACCCAACATGAACCGCACA
GGCTGCCAGCTTATCCCCATCATCAAATTGGAGTGGCATTCTCCCTGGGCTGTGGTGC
CTGTGTTTGTTGCAATATTGGGAATCATCGCCACCACCTTTGTGATCGTGACCTTTGTC
CGCTATAATGACACACCTATCGTGAGGGCTTCAGGACGCGAACTTAGTTACGTGCTCCT
AACGGGGATTTTTCTCTGTTATTCAATCACGTTTTTAATGATTGCAGCACCAGATACAAT
CATATGCTCCTTCCGACGGGTCTTCCTAGGACTTGGCATGTGTTTCAGCTATGCAGCCC
TTCTGACCAAAACAAACCGTATCCACCGAATATTTGAGCAGGGGAAGAAATCTGTCACA
GCGCCCAAGTTCATTAGTCCAGCATCTCAGCTGGTGATCACCTTCAGCCTCATCTCCGT
CCAGCTCCTTGGAGTGTTTGTCTGGTTTGTTGTGGATCCCCCCCACATCATCATTGACT
ATGGAGAGCAGCGGACACTAGATCCAGAGAAGGCCAGGGGAGTGCTCAAGTGTGACA
TTTCTGATCTCTCACTCATTTGTTCACTTGGATACAGTATCCTCTTGATGGTCACTTGTA
CTGTTTATGCCATTAAAACGAGAGGTGTCCCAGAGACTTTCAATGAAGCCAAACCTATT
GGATTTACCATGTATACCACCTGCATCATTTGGTTAGCTTTCATCCCCATCTTTTTTGGT
ACAGCCCAGTCAGCAGAAAAGATGTACATCCAGACAACAACACTTACTGTCTCCATGAG
TTTAAGTGCTTCAGTATCTCTGGGCATGCTCTATATGCCCAAGGTTTATATTATAATTTTT
CATCCAGAACAGAATGTTCAAAAACGCAAGAGGAGCTTCAAGGCTGTGGTGACAGCTG
CCACCATGCAAAGCAAACTGATCCAAAAAGGAAATGACAGACCAAATGGCGAGGTGAA
AAGTGAACTCTGTGAGAGTCTTGAAACCAACACTTCCTCTACCAAGACAACATATATCA
GTTACAGCAATCATTCAATC (SEQ ID NO:7).

The human metabotropic glutamate receptor 8 (mGluR8) may have an amino acid sequence:

MVCEGKRSASCPCFFLLTAKFYWILTMMQRTHSQEYAHSIRVDGDIILGGLFPVHAKGERG
VPCGELKKEKGIHRLEAMLYAIDQINKDPDLLSNITLGVRILDTCSRDTYALEQSLTFVQALIE
KDASDVKCANGDPPIFTKPDKISGVIGAAASSVSIMVANILRLFKIPQISYASTAPELSDNTRY
DFFSRVVPPDSYQAQAMVDIVTALGWNYVSTLASEGNYGESGVEAFTQISREIGGVCIAQS

QKIPREPRPGEFEKIIKRLLETPNARAVIMFANEDDIRRILEAAKKLNQSGHFLWIGSDSWGS
KIAPVYQQEEIAEGAVTILPKRASIDGFDRYFRSRTLANNRRNVWFAEFWEENFGCKLGSH
GKRNSHIKKCTGLERIARDSSYEQEGKVQFVIDAVYSMAYALHNMHKDLCPGYIGLCPRMS
TIDGKELLGYIRAVNFNGSAGTPVTFNENGDAPGRYDIFQYQITNKSTEYKVIGHWTNQLHL
KVEDMQWAHREHTHPASVCSLPCKPGERKKTVKGVPCCWHCERCEGYNYQVDELSCEL
CPLDQRPNMNRTGCQLIPIIKLEWHSPWAVVPVFVAILGIIATTFVIVTFVRYNDTPIVRASGR
ELSYVLLTGIFLCYSITFLMIAAPDTIICSFRRVFLGLGMCFSYAALLTKTNRIHRIFEQGKKSV
TAPKFISPASQLVITFSLISVQLLGVFVWFVVDPPHIIIDYGEQRTLDPEKARGVLKCDISDLSL
ICSLGYSILLMVTCTVYAIKTRGVPETFNEAKPIGFTMYTTCIIWLAFIPIFFGTAQSAEKMYIQT
TTLTVSMSLSASVSLGMLYMPKVYIIIFHPEQNVQKRKRSFKAVVTAATMQSKLIQKGNDRP
NGEVKSELCESLETNTSSTKTTYISYSNHSI (SEQ ID NO:8).

The disclosed designer EVs may be functionalized with GluRs having at least 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity to SEQ ID NO: 2, 4, 6, or 8.

Examples of commercially available constructs for expression of GluRs in donor cells include mGluR4 (NM_001013385; Mouse Tagged ORF Clone; OriGene Technologies, Inc., CAT#: MR210868), mGluR8 (NM_008174; Mouse Tagged ORF Clone; OriGene Technologies, Inc., CAT#: MR211148), Metabotropic Glutamate Receptor 4 (mGluR4) (NM_000841; Human Tagged ORF Clone; OriGene Technologies, Inc., CAT#: RC223673) and Metabotropic Glutamate Receptor 8 (mGluR8) (NM_000845; Human Tagged ORF Clone; OriGene Technologies, Inc., CAT#: RC210813).

The polynucleotides may be delivered to the donor cells for EVs, intracellularly via a gene gun, a microparticle or nanoparticle suitable for such delivery, transfection by electroporation, three-dimensional nanochannel electroporation, a tissue nanotransfection device, a liposome suitable for such delivery, or a deep-topical tissue nanoelectroinjection device. A viral vector can also be used. However, the polynucleotides may also not be delivered virally.

Electroporation is a technique in which an electrical field is applied to cells in order to increase permeability of the cell membrane, allowing cargo (e.g., reprogramming factors) to be introduced into cells. Electroporation is a common technique for introducing foreign DNA into cells.

### Transmembrane Domain

The GluR may be linked to an exosomal or lysosomal transmembrane protein, e.g. expressed as a fusion protein. Design strategies for producing exosomes is described in Liu C, et al. Theranostics. 2019 9(4):1015-1028, which is incorporated by reference for the teaching of transmembrane proteins that can be used to guide fusion proteins into exosomes. Therefore, the transmembrane protein may be selected from the group consisting of CD63, CD9, CD81, CD53, CD82, CD37 (Tetraspanins), Alix (endosome-associated proteins), flotillin-1 (lipid raft-associated protein), TSG101 (Component of the ESCRT-I complex), ARRDC (Arrestin family of protein), Palmitoylated tdTomato (Tandem dimer Tomato fused at NH2-termini with a palmitoylation signal for EV membrane labelling), Lactadherin C1C2 domain (Membrane glycoprotein), EGF VIII (Transmembrane glycoprotein), PDGFR TM domain (Cell surface tyrosine kinase receptor), HIV-1 Nef (mut) (Released in extracellular vesicles), VSVG (Vesicular stomatitis virus glycoprotein), LAMP2B (Lysosome-Associated Membrane Glycoprotein 2), LAMP1 (Lysosome-Associated Membrane Glycoprotein 1), ALIX-1 (Cytosolic protein that associates with MVB by interacting with ESCRT-III subunit SNF7), HSP70 (Heat Shock Protein), HSP90 (Heat Shock Protein), MHC (Anchored in the membrane), SCAMPs (Secretory Carrier-Associated Membrane Protein 18), ApoE (Apolipoprotein E), and WW tag (Recognized by the L-domain-containing protein Ndfip1, resulting in ubiquitination and loading into exosomes).

Also disclosed but not claimed herein are polynucleotides comprising nucleic acid sequences encoding a transmembrane protein suitable for guiding the APC-targeting ligand into an exosome. Examples of this type of proteins include tetraspanins CD9, CD63, and CD81.

Therefore, the transmembrane protein may be CD9 and comprises the amino acid sequence:
MPVKGGTKCIKYLLFGFNFIFWLAGIAVLAIGLWLRFDSQTKSIFEQETNNNNSSFYTGVYILI GAGALMMLVGFLGCCGAVQESQCMLGLFFGFLLVIFAIEIAAAIWGYSHKDEVIKEVQEFYK DTYNKLKTKDEPQRETLKAIHYALNCCGLAGGVEQFISDICPKKDVLETFTVKSCPDAIKEVF DNKFHIIGAVGIGIAVVMIFGMIFSMILCCAIRRNREMV (SEQ ID NO:9), or an amino acid sequence that has at least 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:9. Therefore, the nucleic acid sequence encoding the APC-targeting ligand may be encoded by the nucleic acid sequence: GACCAGCCTACAGCCGCCTGCATCTGTATCCAGCGCCAGGTCCCGCCAGTCCCAGCT GCGCGCGCCCCCCAGTCCCGCACCCGTTCGGCCCAGGCTAAGTTAGCCCTCACCATG CCGGTCAAAGGAGGCACCAAGTGCATCAAATACCTGCTGTTCGGATTTAACTTCATCTT CTGGCTTGCCGGGATTGCTGTCCTTGCCATTGGACTATGGCTCCGATTCGACTCTCAG ACCAAGAGCATCTTCGAGCAAGAAACTAATAATAATAATTCCAGCTTCTACACAGGAGT CTATATTCTGATCGGAGCCGGCGCCCTCATGATGCTGGTGGGCTTCCTGGGCTGCTGC GGGGCTGTGCAGGAGTCCCAGTGCATGCTGGGACTGTTCTTCGGCTTCCTCTTGGTGA TATTCGCCATTGAAATAGCTGCGGCCATCTGGGGATATTCCCACAAGGATGAGGTGATT AAGGAAGTCCAGGAGTTTTACAAGGACACCTACAACAAGCTGAAAACCAAGGATGAGC CCCAGCGGGAAACGCTGAAAGCCATCCACTATGCGTTGAACTGCTGTGGTTTGGCTGG GGGCGTGGAACAGTTTATCTCAGACATCTGCCCCAAGAAGGACGTACTCGAAACCTTC ACCGTGAAGTCCTGTCCTGATGCCATCAAAGAGGTCTTCGACAATAAATTCCACATCAT CGGCGCAGTGGGCATCGGCATTGCCGTGGTCATGATATTTGGCATGATCTTCAGTATG ATCTTGTGCTGTGCTATCCGCAGGAACCGCGAGATGGTCTAGAGTCAGCTTACATCCCT GAGCAGGAAAGTTTACCCATGAAGATTGGTGGGATTTTTTGTTTGTTTGTTTTGTTTTGT TTGTTGTTTGTTGTTTGTTTTTTTGCCACTAATTTTAGTATTCATTCTGCATTGCTAGATAA AAGCTGAAGTTACTTTATGTTTGTCTTTTAATGCTTCATTCAATATTGACATTTGTAGTTG AGCGGGGGGTTTGGTTTGCTTTGGTTTATATTTTTTCAGTTGTTTGTTTTTGCTTGTTATA TTAAGCAGAAATCCTGCAATGAAAGGTACTATATTTGCTAGACTCTAGACAAGATATTGT ACATAAAAGAATTTTTTTGTCTTTAAATAGATACAAATGTCTATCAACTTTAATCAAGTTGT AACTTATATTGAAGACAATTTGATACATAATAAAAAATTATGACAATGTCAAAAAAAAAAA AAAA (SEQ ID NO:10), or a nucleic acid sequence that hybridizes to a nucleic acid sequence consisting of SEQ ID NO:10 under stringent hybridization conditions.

Alternatively, the transmembrane protein may be CD63 and comprises the amino acid sequence:
MAVEGGMKCVKFLLYVLLLAFCACAVGLIAVGVGAQLVLSQTIIQGATPGSLLPVVIIAVGVFL FLVAFVGCCGACKENYCLMITFAIFLSLIMLVEVAAAIAGYVFRDKVMSEFNNNFRQQMENY PKNNHTASILDRMQADFKCCGAANYTDWEKIPSMSKNRVPDSCCINVTVGCGINFNEKAIH KEGCVEKIGGWLRKNVLVVAAAALGIAFVEVLGIVFACCLVKSIRSGYEVM (SEQ ID NO:11), or an amino acid sequence that has at least 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:11. Therefore, the nucleic acid sequence encoding the APC-targeting ligand may be encoded by the nucleic acid sequence:
ATGGCGGTGGAAGGAGGAATGAAATGTGTGAAGTTCTTGCTCTACGTCCTCCTGCTGG CCTTTTGCGCCTGTGCAGTGGGACTGATTGCCGTGGGTGTCGGGGCACAGCTTGTCCT GAGTCAGACCATAATCCAGGGGGCTACCCCTGGCTCTCTGTTGCCAGTGGTCATCATC GCAGTGGGTGTCTTCCTCTTCCTGGTGGCTTTTGTGGGCTGCTGCGGGGCCTGCAAGG AGAACTATTGTCTTATGATCACGTTTGCCATCTTTCTGTCTCTTATCATGTTGGTGGAGG TGGCCGCAGCCATTGCTGGCTATGTGTTTAGAGATAAGGTGATGTCAGAGTTTAATAAC AACTTCCGGCAGCAGATGGAGAATTACCCGAAAAACAACCACACTGCTTCGATCCTGG ACAGGATGCAGGCAGATTTTAAGTGCTGTGGGGCTGCTAACTACACAGATTGGGAGAA AATCCCTTCCATGTCGAAGAACCGAGTCCCCGACTCCTGCTGCATTAATGTTACTGTGG GCTGTGGGATTAATTTCAACGAGAAGGCGATCCATAAGGAGGGCTGTGTGGAGAAGAT TGGGGGCTGGCTGAGGAAAAATGTGCTGGTGGTAGCTGCAGCAGCCCTTGGAATTGCT TTTGTCGAGGTTTTGGGAATTGTCTTTGCCTGCTGCCTCGTGAAGAGTATCAGAAGTGG CTACGAGGTGATG (SEQ ID NO:12), or a nucleic acid sequence that hybridizes to a nucleic acid sequence consisting of SEQ ID NO:12 under stringent hybridization conditions.

Therefore, the transmembrane protein may be CD81 and comprises the amino acid sequence:
MGVEGCTKCIKYLLFVFNFVFWLAGGVILGVALWLRHDPQTTNLLYLELGDKPAPNTFYVGI YILIAVGAVMMFVGFLGCYGAIQESQCLLGTFFTCLVILFACEVAAGIWGFVNKDQIAKDVKQ FYDQALQQAVVDDDANNAKAVVKTFHETLDCCGSSTLTALTTSVLKNNLCPSGSNIISNLFK EDCHQKIDDLFSGKLYLIGIAAIVVAVIMIFEMILSMVLCCGIRNSSVY (SEQ ID NO:13), or an amino acid sequence that has at least 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% sequence identity to SEQ ID NO:13. Therefore, the nucleic acid sequence encoding the APC-targeting ligand may be encoded by the nucleic acid sequence:
GGCCAGAGAGCGAGCGCGCAACGGCGGCGACGGCGGCGACCCCACCGCGCATCCTG CCAGGCCTCCGGCGCCCAGCGCCCCACGCGCCCCCGCGCCCCCGCGCCCCCGCGCC CCTTTCTTCGCGCCCCCGCCCCTCGGCCCGCCAGGCCCCCTTGCCGGCCACCCGCCA GGCCCCGCGCCGGCCCGCCCGCCGCCCAGGACCGGCCCGCGCCCCGCAGGCCGCC CGCCGCCCGCGCCGCCATGGGAGTGGAGGGCTGCACCAAGTGCATCAAGTACCTGCT CTTCGTCTTCAATTTCGTCTTCTGGCTGGCTGGAGGCGTGATCCTGGGTGTGGCCCTGT GGCTCCGCCATGACCCGCAGACCACCAACCTCCTGTATCTGGAGCTGGGAGACAAGCC CGCGCCCAACACCTTCTATGTAGGCATCTACATCCTCATCGCTGTGGGCGCTGTCATGAT GTTCGTTGGCTTCCTGGGCTGCTACGGGGCCATCCAGGAATCCCAGTGCCTGCTGGGG ACGTTCTTCACCTGCCTGGTCATCCTGTTTGCCTGTGAGGTGGCCGCCGGCATCTGGG GCTTTGTCAACAAGGACCAGATCGCCAAGGATGTGAAGCAGTTCTATGACCAGGCCCTA CAGCAGGCCGTGGTGGATGATGACGCCAACAACGCCAAGGCTGTGGTGAAGACCTTCC ACGAGACGCTTGACTGCTGTGGCTCCAGCACACTGACTGCTTTGACCACCTCAGTGCT CAAGAACAATTTGTGTCCCTCGGGCAGCAACATCATCAGCAACCTCTTCAAGGAGGACT GCCACCAGAAGATCGATGACCTCTTCTCCGGGAAGCTGTACCTCATCGGCATTGCTGCC ATCGTGGTCGCTGTGATCATGATCTTCGAGATGATCCTGAGCATGGTGCTGTGCTGTGG CATCCGGAACAGCTCCGTGTACTGAGGCCCCGCAGCTCTGGCCACAGGGACCTCTGCA GTGCCCCCTAAGTGACCCGGACACTTCCGAGGGGGCCATCACCGCCTGTGTATATAAC GTTTCCGGTATTACTCTGCTACACGTAGCCTTTTTACTTTTGGGGTTTTGTTTTTGTTCTG AACTTTCCTGTTACCTTTTCAGGGCTGACGTCACATGTAGGTGGCGTGTATGAGTGGAG ACGGGCCTGGGTCTTGGGGACTGGAGGGCAGGGGTCCTTCTGCCCTGGGGTCCCAG GGTGCTCTGCCTGCTCAGCCAGGCCTCTCCTGGGAGCCACTCGCCCAGAGACTCAGC TTGGCCAACTTGGGGGGCTGTGTCCACCCAGCCCGCCCGTCCTGTGGGCTGCACAGC TCACCTTGTTCCCTCCTGCCCCGGTTCGAGAGCCGAGTCTGTGGGCACTCTCTGCCTT CATGCACCTGTCCTTTCTAACACGTCGCCTTCAACTGTAATCACAACATCCTGACTCCGT CATTTAATAAAGAAGGAACATCAGGCATGCTA SEQ ID NO:14), or a nucleic acid sequence that hybridizes to a nucleic acid sequence consisting of SEQ ID NO:14 under stringent hybridization conditions.

### Cargo

The disclosed GluR-decorated EVs can be used to deliver a wide variety of molecular cargo to the injured regions of the CNS. They can, for example, be used to selectively deliver proangiogenic, proneurogenic, anti-inflammatory, or neuroprotective molecular cargo to the brain, to boost vasculogenic and neurogenic repair processes, as well as modulating the inflammatory response after injury.

The GluR-decorated EVs may be coupled with other nanostructures (e.g., gold nanoparticles, magnetic nanoparticles, quantum dots, carbon nanotubes) either via encapsulation of chemical coupling (e.g., surface decoration) to enable additional therapeutic (e.g., hyperthermia, phototherapy, etc.) or theranostics applications.

In some embodiments, the disclosed GluR-decorated EVs can be used for diagnostic applications to achieve targeted delivery of a wide array of diagnostic agents, including molecular probes for nucleic acids or proteins, contrast/imaging agents (e.g., magnetic nanoparticles, plasmonic gold nanoparticles, quantum dots), etc.

### Pharmaceutical Compositions

Disclosed is a pharmaceutical composition containing therapeutically effective amounts of one or more of the disclosed EVs and a pharmaceutically acceptable carrier. Formulations containing the disclosed EVs may take the form of liquid, solid, semi-solid or lyophilized powder forms, such as, for example, solutions, suspensions, emulsions, sustained-release formulations, tablets, capsules, powders, suppositories, creams, ointments, lotions, aerosols, patches or the like, preferably in unit dosage forms suitable for simple administration of precise dosages.

Pharmaceutical compositions typically include a conventional pharmaceutical carrier and/or excipient and may additionally include other medicinal agents, carriers, adjuvants, additives and the like. The weight percentage ratio of the EVs to the one or more excipients can be between about 20:1 to about 1:60, or between about 15:1 to about 1:45, or between about 10:1 to about 1:40, or between about 9:1, 8:1, 7:1, 6:1, 5:1, 4:1, 3:1, 2:1 or 1:1 to about 1:2, 1:3, 1:4, 1:5, 1:6, 1:7, 1:8, 1:9, 1:10, 1:15, 1:20, 1:25, 1:30, or 1:35, and preferably is about 20:1, 19:1, 18:1, 17:1, 16:1, 15:1, 14:1, 13:1, 12:1, 11:1, 10:1, 9:1, 8:1, 7:1, 6:1 or 5:1. The disclosed composition may comprise between about 1 µg to about 1 g or more of total EVs, about 500 µg about 500 mg, about 1 mg to about 500 mg of total EVs, about 5 to about 500 mg, about 10 to about 500 mg, about 25 to about 500 mg, about 50 mg to about 350 mg, about 75 mg to about 450 mg, about 50 mg to about 450 mg, or about 75 mg to about 325 mg or about 100 mg to about 650 mg of total EVs and may optionally contain one or more suitable pharmaceutical carriers, additives and/or excipients.

An injectable composition for parenteral administration (e.g. intravenous, intramuscular, intrathecal intracerebrospinal fluid, or intranasal), will typically contain the EVs and optionally additional components in a suitable i.v. solution, such as sterile physiological salt solution. The composition may also be formulated as a suspension in an aqueous emulsion.

Liquid compositions can be prepared by dissolving or dispersing the pharmaceutical composition comprising the EVs, and optional pharmaceutical adjuvants, in a carrier, such as, for example, aqueous saline, aqueous dextrose, glycerol, or ethanol, to form a solution or suspension. For use in an oral liquid preparation, the composition may be prepared as a solution, suspension, emulsion, or syrup, being supplied either in liquid form or a dried form suitable for hydration in water or normal saline. In the case of intranasal, intratracheal or intrapulmonary administration, the compositions may be provided as liquid composition which can be sprayed into the nose, trachea and/or lungs.

For oral administration, such excipients include pharmaceutical grades of mannitol, lactose, starch, magnesium stearate, sodium saccharine, talcum, cellulose, glucose, gelatin, sucrose, magnesium carbonate, and the like. If desired, the composition may also contain minor amounts of non-toxic auxiliary substances such as wetting agents, emulsifying agents, or buffers.

When the composition is employed in the form of solid preparations for oral administration, the preparations may be tablets, granules, powders, capsules or the like. In a tablet formulation, the composition is typically formulated with additives, e.g. an excipient such as a saccharide or cellulose preparation, a binder such as starch paste or methyl cellulose, a filler, a disintegrator, and other additives typically used in the manufacture of medical preparations.

Methods for preparing such dosage forms are known or are apparent to those skilled in the art; for example, see Remington's Pharmaceutical Sciences (17th Ed., Mack Pub. Co. 1985). The composition to be administered will contain a quantity of the selected compound in a pharmaceutically effective amount for therapeutic use in a biological system, including a patient or subject according to the present invention.

Intravenous formulations can comprise the EVs described herein, an isotonic medium and one or more substances preventing aggregation of the EVs. Example intravenous/ intrathecal/ intracerebrospinal fluid formulations may contain saline solutions (e.g. normal saline (NS); about 0.91% w/v of NaCl, about 300 mOsm/L) and/or dextrose 4% in 0.18% saline, and optionally 1%, 2% or 3% human serum albumin. In addition, the EVs may be disrupted to obtain the contents and the contents used in compositions according to the present invention.

Formulations of the invention may comprise about 50 ng EVs/ml intravenous/intrathecal/intracerebrospinal fluid medium, including about 100 ng, 200 ng, 300 ng, 400 ng, 500 ng, 600 ng, 700 ng, 800 ng, 900 ng, 1.0 µg, 1.5 µg, 2.0 µg, 2.5 µg, 3.0 µg, 5.0 µg, 10.0, 15.0 µg, 20.0 µg, 100 µg, or more EVs/ml intravenous/intrathecal/intracerebrospinal fluid medium for use in treating spinal cord injury, stroke, traumatic brain injury and/or neurodegenerative diseases.

Intravenous formulations may comprise about 0.1 µg EVs/ml medium, about 0.2 µg EVs/ml intravenous medium, about 0.3 µg EVs/ml intravenous medium, about 0.4 µg EVs/ml intravenous medium, about 0.5 µg EVs/ml intravenous medium, about 0.6 µg EVs/ml intravenous medium, about 0.7 µg EVs/ml intravenous medium, about 0.8 µg EVs/ml intravenous medium, about 0.9 µg EVs/ml intravenous medium, about 1.0 µg EVs/ml intravenous medium, about 1.5 µg EVs/ml intravenous medium, about 2.0 µg EVs/ml intravenous medium, about 2.5 µg EVs/ml intravenous medium, such as at least e.g. about 3.0 µg EVs/ml intravenous medium, such as e.g. at least about 5.0 µg EVs/ml intravenous medium, about 10.0 µg EVs/ml intravenous medium, 15.0 µg EVs/ml intravenous medium or about 20.0 µg or more EVs/ml intravenous medium.

The pharmaceutical composition may be in a dosage form comprising at least 25 mg of EVs, at least 50 mg of EVs, at least 60 mg of EVs, at least 75 mg of EVs, at least 100 mg of EVs, at least 150 mg of EVs, at least 200 mg of EVs, at least 250 mg of EVs, at least 300 mg of EVs, about 350 mg of EVs, about 400 mg of EVs, about 500 mg of EVs, about 750 mg of EVs, about 1g (1,000mg) or more of EVs, alone or in combination with a therapeutically effective amount of at least one additional bioactive agent, which agent may be useful in the treatment of spinal cord injury, stroke, traumatic brain injury and/or neurodegenerative disease. The pharmaceutical composition may comprise between about 10 mg to about 750 mg, about 25 mg to about 650 mg, or between about 30 mg to about 500 mg, or about 35 mg to about 450 mg, most often about 50 to about 500 mg of EVs.

An intravenous formulation may comprise the EVs described herein, an isotonic medium, and one or more substances preventing aggregation of the EVs. Intravenous formulations may therefore contain saline solutions (e.g. normal saline (NS); about 0.91% w/v of NaCl, about 300 mOsm/L) and/or dextrose 4% in 0.18% saline, and optionally 1%, 2% or 3% human serum albumin.

The composition comprising the disclosed EVs may further comprise one more neurotrophic agents. The composition can further comprises one or more agents selected from the group consisting of leukemia inhibitory factor (LIF), brain-derived neurotrophic factor (BDNF), epidermal growth factor receptor (EGF), basic fibroblast growth factor (bFGF), FGF-6, glial-derived neurotrophic factor (GDNF), granulocyte colony-stimulating factor (GCSF), hepatocyte growth factor (HGF), IFN-γ, insulin-like growth factor binding protein (IGFBP-2), IGFBP-6, IL-1ra, IL-6, IL-8, monocyte chemotactic protein (MCP-1), mononuclear phagocyte colony-stimulating factor (M-CSF), neurotrophic factors (NT3), tissue inhibitor of metalloproteinases (TIMP-1), TIMP-2, tumor necrosis factor (TNF-β), vascular endothelial growth factor (VEGF), VEGF-D, urokinase plasminogen activator receptor (uPAR), bone morphogenetic protein 4 (BMP4), IL1-a, IL-3, leptin, stem cell factor (SCF), stromal cell-derived factor-1 (SDF-1), platelet derived growth factor-BB (PDGFBB), transforming growth factors beta (TGFβ-1) and TGFβ-3.

The disclosed EVs may be contained in or on a biocompatible scaffold, such as a hydrogel. Suitable hydrogels include temperature dependent hydrogels that solidify or set at body temperature, e.g., PLURONICS^{™}; hydrogels crosslinked by ions, e.g., sodium alginate; hydrogels set by exposure to either visible or ultraviolet light, e.g., polyethylene glycol polylactic acid copolymers with acrylate end groups; and hydrogels that are set or solidified upon a change in pH, e.g., TETRONICS^{™}. Examples of materials that can be used to form these different hydrogels include polysaccharides such as alginate, polyphosphazenes, and polyacrylates, which are cross-linked ionically, or block copolymers such as PLURONICS^{™} (also known as POLOXAMERS^{™}), which are poly(oxyethylene)-poly(oxypropylene) block polymers solidified by changes in temperature, or TETRONICS^{™} (also known as POLOXAMINES^{™}), which are poly(oxyethylene)-poly(oxypropylene) block polymers of ethylene diamine solidified by changes in pH.

Suitable hydrogels also include undefined extracellular matrix derived hydrogels that originated from tissues including but not limited to bladder intestine, blood and brain.

The disclosed EVs may be contained in or on a biocompatible scaffold comprising collagen, fibrin, silk, agarose, alginate, hyaluronan, chitosan, a biodegradable polyester such as polylactic-co-glycolic acid, polylacic acid, or polyglycolic acid, polyethylene glycol, polyvinylpyrrolidone, polyethersulfone, a peptide-based biomaterial, glycose amino glycan, fibronectin, laminin, or any combination thereof.

In some cases, the hydrogel is produced by cross-linking the anionic salt of alginic acid, a carbohydrate polymer isolated from seaweed, with ions, such as calcium cations. The strength of the hydrogel increases with either increasing concentrations of calcium ions or alginate. For example, U.S. Pat. No. 4,352,883 describes the ionic cross-linking of alginate with divalent cations, in water, at room temperature, to form a hydrogel matrix.

EVs are mixed with an alginate solution, the solution is delivered to an already implanted support structure and then solidifies in a short time due to the presence in vivo of physiological concentrations of calcium ions. Alternatively, the solution is delivered to the support structure prior to implantation and solidified in an external solution containing calcium ions.

In general, these polymers are at least partially soluble in aqueous solutions, e.g., water, or aqueous alcohol solutions that have charged side groups, or a monovalent ionic salt thereof. There are many examples of polymers with acidic side groups that can be reacted with cations, e.g., poly(phosphazenes), poly(acrylic acids), and poly(methacrylic acids). Examples of acidic groups include carboxylic acid groups, sulfonic acid groups, and halogenated (preferably fluorinated) alcohol groups. Examples of polymers with basic side groups that can react with anions are poly(vinyl amines), poly(vinyl pyridine), and poly(vinyl imidazole).

Polyphosphazenes are polymers with backbones consisting of nitrogen and phosphorous atoms separated by alternating single and double bonds. Each phosphorous atom is covalently bonded to two side chains. Polyphosphazenes that can be used have a majority of side chains that are acidic and capable of forming salt bridges with di- or trivalent cations. Examples of acidic side chains are carboxylic acid groups and sulfonic acid groups.

Bioerodible polyphosphazenes have at least two differing types of side chains, acidic side groups capable of forming salt bridges with multivalent cations, and side groups that hydrolyze under in vivo conditions, e.g., imidazole groups, amino acid esters, glycerol, and glucosyl. Bioerodible or biodegradable polymers, i.e., polymers that dissolve or degrade within a period that is acceptable in the desired application (usually in vivo therapy), will degrade in less than about five years and most preferably in less than about one year, once exposed to a physiological solution of pH 6-8 having a temperature of between about 25° C. and 38° C. Hydrolysis of the side chain results in erosion of the polymer. Examples of hydrolyzing side chains are unsubstituted and substituted imidizoles and amino acid esters in which the side chain is bonded to the phosphorous atom through an amino linkage.

Methods for synthesis and the analysis of various types of polyphosphazenes are described in U.S. Pat. Nos. 4,440,921, 4,495,174, and 4,880,622. Methods for the synthesis of the other polymers described above are known to those skilled in the art. See, for example Concise Encyclopedia of Polymer Science and Engineering, J. I. Kroschwitz, editor (John Wiley and Sons, New York, N.Y., 1990). Many polymers, such as poly(acrylic acid), alginates, and PLURONICS^{™}, are commercially available.

Water soluble polymers with charged side groups are cross-linked by reacting the polymer with an aqueous solution containing multivalent ions of the opposite charge, either multivalent cations if the polymer has acidic side groups, or multivalent anions if the polymer has basic side groups. Cations for cross-linking the polymers with acidic side groups to form a hydrogel include divalent and trivalent cations such as copper, calcium, aluminum, magnesium, and strontium. Aqueous solutions of the salts of these cations are added to the polymers to form soft, highly swollen hydrogels.

Anions for cross-linking the polymers to form a hydrogel include divalent and trivalent anions such as low molecular weight dicarboxylate ions, terepthalate ions, sulfate ions, and carbonate ions. Aqueous solutions of the salts of these anions are added to the polymers to form soft, highly swollen hydrogels, as described with respect to cations.

For purposes of preventing the passage of antibodies into the hydrogel, but allowing the entry of nutrients, a useful polymer size in the hydrogel is in the range of between 10,000 D and 18,500 D.

Temperature-dependent, or thermosensitive, hydrogels have so-called "reverse gelation" properties, i.e., they are liquids at or below room temperature, and gel when warmed to higher temperatures, e.g., body temperature. Thus, these hydrogels can be easily applied at or below room temperature as a liquid and automatically form a semi-solid gel when warmed to body temperature. As a result, these gels are especially useful when the support structure is first implanted into a patient, and then filled with the hydrogel-EV composition. Examples of such temperature-dependent hydrogels are PLURONICS^{™} (BASF-Wyandotte), such as polyoxyethylene-polyoxypropylene F-108, F-68, and F-127, poly(N-isopropylacrylamide), and N-isopropylacrylamide copolymers.

These copolymers can be manipulated by standard techniques to affect their physical properties such as porosity, rate of degradation, transition temperature, and degree of rigidity. For example, the addition of low molecular weight saccharides in the presence and absence of salts affects the lower critical solution temperature (LCST) of typical thermosensitive polymers. In addition, when these gels are prepared at concentrations ranging between 5 and 25% (W/V) by dispersion at 4°C., the viscosity and the gel-sol transition temperature are affected, the gel-sol transition temperature being inversely related to the concentration.

U.S. Pat. No. 4,188,373 describes using PLURONIC^{™} polyols in aqueous compositions to provide thermal gelling aqueous systems. U.S. Pat. Nos. 4,474,751, '752, '753, and 4,478,822 describe drug delivery systems which utilize thermosetting polyoxyalkylene gels; with these systems, both the gel transition temperature and/or the rigidity of the gel can be modified by adjustment of the pH and/or the ionic strength, as well as by the concentration of the polymer.

pH-dependent hydrogels are liquids at, below, or above specific pH values, and gel when exposed to specific pHs, e.g., 7.35 to 7.45, the normal pH range of extracellular fluids within the human body. Thus, these hydrogels can be easily delivered to an implanted support structure as a liquid and automatically form a semi-solid gel when exposed to body pH. Examples of such pH-dependent hydrogels are TETRONICS^{™} (BASF-Wyandotte) polyoxyethylene-polyoxypropylene polymers of ethylene diamine, poly(diethyl aminoethyl methacrylate-g-ethylene glycol), and poly(2-hydroxymethyl methacrylate). These copolymers can be manipulated by standard techniques to affect their physical properties.

Hydrogels that are solidified by either visible or ultraviolet light can be made of macromers including a water soluble region, a biodegradable region, and at least two polymerizable regions as described in U.S. Pat. No. 5,410,016. For example, the hydrogel can begin with a biodegradable, polymerizable macromer including a core, an extension on each end of the core, and an end cap on each extension. The core is a hydrophilic polymer, the extensions are biodegradable polymers, and the end caps are oligomers capable of cross-linking the macromers upon exposure to visible or ultraviolet light, e.g., long wavelength ultraviolet light.

Examples of such light solidified hydrogels include polyethylene oxide block copolymers, polyethylene glycol polylactic acid copolymers with acrylate end groups, and 10K polyethylene glycol-glycolide copolymer capped by an acrylate at both ends. As with the PLURONIC^{™} hydrogels, the copolymers comprising these hydrogels can be manipulated by standard techniques to modify their physical properties such as rate of degradation, differences in crystallinity, and degree of rigidity.

### Medical uses

Application of the disclosed GluR-functionalized designer EVs can be translated to a wide variety of conditions involving CNS injuries. According to the CDC, stroke is the leading cause of long-term disability in the US, with more than 795,000 cases reported yearly and an estimated annual cost of $34 billion. Ischemic strokes, which have the highest incidence rate (-87%), result in significant cellular death (e.g. vascular, neuronal) and inflammation. Currently, there is only one FDA-approved treatment for ischemic stroke, and it is an acute measure limited only to quickly restoring blood flow. However, its implementation is restricted to the first 4.5 hours, and therefore more than 95% of patients are not eligible for it. As such, there is still a need for effective therapies to attenuate tissue damage and aid repair post-stroke.

Therefore, also disclosed is a composition containing a population of the designer EVs disclosed herein for use in treating a subject with a CNS injury comprising administering to the subject an effective amount of the composition disclosed herein.

In some embodiments, the CNS injury is a spinal cord injury, stroke, traumatic brain injury or a neurodegenerative disease, such as Alzheimer's disease, Parkinson's disease, a Parkinson's-related disorder, Huntington's disease, prion disease, motor neuron disease (MND), spinocerebellar ataxia (SCA) or spinal muscular atrophy (SMA), or multiple sclerosis (MS).

The designer EV may serve as glutamate scavenging agents, helping to decrease the noxious concentration of free glutamate in injured regions of the brain, and aiding brain tissue recovery. In some embodiments, the designer EVs contain therapeutic cargo, such as a proangiogenic, proneurogenic, or anti-infalmmatory molecular cargo to boost vasculogenic and neurogenic repair processes, as well as modulating the inflammatory response after injury.

The term "spinal cord injury" is used to describe a spinal cord injury which results in a temporary or permanent change in the normal motor, autonomic or sensory function of the cord. The damage often results from physical trauma, such as sports injuries, slip and fall accidents or motor vehicular accidents but can also result from diseases such as spina bifida, Friedrich's ataxis and/or transverse myelitis. Injury to the spinal cord resulting in a loss of function does not have to be the result of complete severing of the spinal cord. Depending on where the spinal cord and its nerve roots are damaged, the symptoms and degree of injury can vary widely, from pain to incontinence to paralysis. Spinal cord injuries are described at various levels of incomplete to complete injury, resulting in a total loss of function. The spinal cord injury can result in paraplegia or tetraplegia.

Traditional treatment of spinal cord injuries starts with stabilizing the spine and controlling inflammation associated with the spinal cord damage to prevent further damage. Other interventions can vary widely depending on the location and extent of the injury. In many cases, using conventional therapy, spinal cord injuries require substantial, long-term physical therapy and rehabilitation, especially if the injury interferes with activities of daily life.

Spinal cord injury can be classified into three types based on its cause: mechanical forces, toxic, and ischemic, from lack of blood flow. Spinal cord damage can also be divided into primary and secondary injury. Primary injury is caused by the cell death that occurs immediately in the original injury (physical trauma, exposure to toxins, or ischemia), and secondary injury is caused by the resultant cascades that are caused by the original insult and cause further tissue damage. These secondary injury pathways include inflammation, swelling, neurotransmitter deficiencies/imbalances, the results of ischemia and cell suicide. The present invention may be used to treat all forms of spinal cord injury, including complete and incomplete injuries, ischemia, spinal cord injury without radiographic abnormality, central cord syndrome, anterior cord syndrome, Brown-Séquard syndrome, posterior cord syndrome, tabes dorsalis and conus medullaris, among others.

The term "stroke" is used to describe a cerebrovascular accident (CVA), cerebrovascular insult (CVI), or brain attack, occurs when poor blood flow to the brain results in cell death. There are two main types of stroke: ischemic, due to lack of blood flow, and hemorrhagic, due to bleeding. Both of these types of stroke result in part of the brain not functioning properly. Signs and symptoms of a stroke may include an inability to move or feel on one side of the body, problems understanding or speaking, a sense of spinning, or loss of vision to one side, among others. Signs and symptoms often appear soon after the stroke has occurred. If symptoms last less than one or two hours it is known as a transient ischemic attack. Hemorrhagic strokes may also be associated with a severe headache. The symptoms of a stroke can be permanent. Long term complications of stroke may include pneumonia or loss of bladder control. The main risk factor for stroke is high blood pressure. Other risk factors include tobacco smoking, obesity, high blood cholesterol, diabetes mellitus, previous transient ischemic attack (TIA), and atrial fibrillation, among others. An ischemic stroke is typically caused by blockage of a blood vessel. A hemorrhagic stroke is caused by bleeding either directly into the brain or into the space surrounding the brain. Bleeding may occur due to a brain aneurysm. Both ischemic and hemorrhagic stroke are treated pursuant to the present invention.

The term "traumatic brain injury" (TBI) is used to describe an injury to the brain caused by movement of the brain within the skill or an injury to the brain caused by a foreign object. Causes of TBI may include falls, a motor vehicle crash or being struck by or with an object. TBI may also be caused by a penetrating object- an injury to the brain caused by a foreign object entering the skull. Causes may include firearm injuries or being struck with a sharp object. TBI may cause a concussion, a period of unconsciousness (coma) or amnesia. TBI may impair one or more of cognitive function (e.g., attention and memory), motor function (e.g., extremity weakness, impaired coordination and balance), sensation (e.g., hearing, vision, impaired perceptin and touch and emotion (e.g., depression, anxiety, aggression, impulse control, personality changes).

The term "neurodegenerative disease" is used throughout the specification to describe a disease which is caused by damage to the central nervous system and which damage can be reduced and/or alleviated through transplantation of neural cells according to the present invention to damaged areas of the brain and/or spinal cord of the patient. Exemplary neurodegenerative diseases which may be treated using the compositions according to the present invention include for example, Parkinson's disease, Huntington's disease, amyotrophic lateral sclerosis (Lou Gehrig's disease), Alzheimer's disease, lysosomal storage disease ("white matter disease" or glial/demyelination disease, as described, for example by Folkerth, J. Neuropath. Exp. Neuro., 58, 9, Sep., 1999), Tay Sachs disease (beta hexosamimidase deficiency), other genetic diseases, multiple sclerosis, brain injury or trauma caused by ischemia, accidents, environmental insult, etc., spinal cord damage, ataxia and alcoholism. In addition, the present invention may be used to reduce and/or eliminate the effects on the central nervous system of a stroke or a heart attack in a patient, which is otherwise caused by lack of blood flow or ischemia to a site in the brain of said patient or which has occurred from physical injury to the brain and/or spinal cord. The term neurodegenerative diseases also includes neurodevelopmental disorders including for example, autism and related neurological diseases such as schizophrenia, among numerous others.

The herein disclosed compositions, including pharmaceutical composition, may be administered in a number of ways depending on whether local or systemic treatment is desired, and on the area to be treated.

Treating subjects involves administration of a pharmaceutical composition comprising an effective amount of EVs described herein and optionally at least one additional bioactive (e.g. an agent which is useful in the treatment of a neurodegenerative disease, stroke and/or spinal cord injury) agent. For example, the compositions could be formulated so that a therapeutically effective dosage of between about 0.01, 0.1, 1, 5, 10, 15, 20, 25, 30 , 35, 40, 45, 50, 55, 60, 65, 70, 75, 80, 85, 90 or 100 mg/kg of patient/day or greater than 100, 110, 120, 130, 140, 150, 160, 170, 180, 190 or 200 mg/kg of the disclosed EVs can be administered to a patient receiving these compositions.

The dose of EVs administered to a subject can be less than 10 µg, less than 25 µg, less than 50 µg, less than 75 µg, less than 0.10 mg, less than 0.25 mg, less than 0.5 mg, less than 1 mg, less than 2.5 mg, less than 5 mg, less than 10 mg, less than 15 mg, less than 20 mg, less than 50 mg, less than 75 mg, less than 100 mg, less than 500 mg, less than 750 mg, less than 1 g or more than 1 g. Administration may be by numerous routes of administration, but intravenous, intrathecal, intranasal and/or intracerebrospinal fluid are often used as routes of administration.

The disclosed EVs may be administered within 24 after a stroke or trauma. However, the EVs may also be administered at least 1, 2, 3, or 4 weeks after a stroke or trauma. The disclosed EVs may be administered in multiple doses 1, 2, 3, or more days apart. In some cases, such as cases of neurodegenerative disease, the EVs are administered continuously (e.g., once every 1, 2, 3, or 4 weeks) over the course of the disease.

EVs may be loaded with small molecules, antisense oligonucleotides, siRNAs, peptides, proteins or antibodies that target, peptides or peptide *translation products which are involved in neurodegenerative processes.*

The disclosed EVs may be loaded with additional bioactive agents or coadministered with additional bioactive agents, especially agents which are useful in the treatment of neurodegenerative diseases.

The term "coadministered", "coadministration" or "combination therapy" is used to describe a therapy in which at least two active compounds/compositions in effective amounts are used to treat neural injury and/or a neurodegenerative disease. Although the term co-administration preferably includes the administration of EVs and at least one additional active compound to the subject at the same time, it is not necessary that the compounds/compositions be administered to the patient simultaneously, only that effective amounts of the individual compounds/compositions be present in the patient at the same time. Thus, the term co-administration includes an administration in which the EVs and the bioactive agent(s) are administered at approximately the same time (contemporaneously), or from about one to several minutes to about eight hours, about 30 minutes to about 6 hours, about an hour to about 4 hours, or even much earlier than the other compound/composition as otherwise described herein including up to a day or substantially more.

Agents which may be loaded or coadministered along with EVs may include, for example aricept, namenda, donepezil, excelon, razadyne, glantamine, rivastigmine, memantine, ergoloid, namzaric and mixtures thereof for Alzheimer's disease, biperiden, apomorphine, trihexyphenidyl, carbidopa/levodopa, rasagline, belladona, levodopa, benztropine, entacapone, selegiline, rivastigmine, pramipexole, rotigotine, bromocriptine, pergolide, ropinirole, carbidopa/entacapone/levodopa, amantadine, tolcopone, trihexiphenidyl and mixtures thereof, for Parkinson's disease, tetrabenazine, haloperidol, chlorpromazine, olanzapine, fluoxetine, sertraline, nortriptyline, benzodiazpines, paroxetine, venlafaxin, beta-blockers, lithium, valproate, carbamazepine, botulinum toxin and mixtures thereof for the treatment of Huntington's disease, anticholinergic drugs, anticonvulsants, antidepressants, benzodiazepines, decongestants, muscle relaxants, pain medications, stimulants and mixtures thereof for the treatment of motor neuron disease, selective serotonin reuptake inhibitors (SSRI's), selective norepinephrine-serotoning reuptake inhibitors (SNRI's), acetazolamide, baclofen, clonazepam, flunarizine, gabapentin, meclizine, memantine, ondansetron, scopolamine, modafinil, armodafinil, amantadine, atomoxetine, buproprion, carnitine, creatine, modafinil, armodafinil, pyrudistigmine, selegiline, venlafaxine, desvenlafaxine, buspirone, riluzole, verenicline, memantine, baclofen, tizanidine, cymbalta, lyrica, acetazolamide, carbamazepine, clonazepam, isoniazid, droxidopa, ephedrine, fludrocortisones, midodrine, levodopa, pramipexole, fluoxetine, n-acetylcysteine, baclofen, dantrolene sodium, diazepam, ropinirole, tizanidine, trihexylphenidyl, clonazepine, flunarazine, levetiracetam, primidone, topiramate, valproic acid, phenytoin, 4-aminopyridine and mixtures thereof for the treatment of spinocerebellar ataxia and riluzole for the treatment of spinal muscular atrophy. Agents for the treatment of stroke include salicylates, such as aspirin, a thrombolytic agent (alteplase) and a platelet aggregation inhibitor (clopidogrel), among others.

More generally, non-steroidal anti-inflammatory drugs (NSAIDS) and other anti-inflammatory agents may be used in the treatment of neurodegenerative diseases as described herein.

The activities of EVs described herein can be evaluated by methods known in the art. The amount of EVs required for use in treatment can vary not only with the particular cell from which the EVs are prepared, but also with the route of administration, the nature of the condition being treated and the age and condition of the patient and can be ultimately at the discretion of the attendant physician or clinician. In general, however, a dose can be in the range of from about 0.01 mg/kg to about 10 mg/kg of body weight per day.

Identifying EVs useful for treating a spinal cord injury, stroke, traumatic brain injury and/or a neurodegenerative disease which occurs by modulating the activity and expression of a disease-related protein and biologically active fragments thereof can be made by screening EV activity in any of a variety of screening techniques. The screening can be made for whole EVs or their contents. Fragments employed in such screening tests may be free in solution, affixed to a solid support, borne on a cell surface, or located intracellularly. The blocking or reduction of biological activity or the formation of binding complexes between the disease-related protein, the EVs and/or one or more components of the EVs may be measured by methods available in the art.

### EXAMPLES

### Example 1:

Experiments in which mGluR4/mGluR8-decorated EVs were delivered intranasally into C57BL6 mice suggest that such decoration strategy favored homing to brain tissue. In vitro studies with primary co-cultures of glia and neurons further indicate selective uptake of mGluR4/mGluR8-decorated EVs by neurons only shortly after exposure (i.e. approximately 8 hours), followed by more widespread uptake by neurons and glia alike after approximately 24 hours. While mGluR4/mGluR8 decoration seems to help to narrow down the range of action of these EVs to brain tissue (minimizing off-target side effects), we believe that neuronal tissue injury, such as stroke events, will help to further guide mGluR4/mGluR8-decorated EVs primarily to stroke-affected areas due to the fact that stroke insults lead to a marked increase in extracellular glutamate associated with profuse neuro- and excitotoxicity.

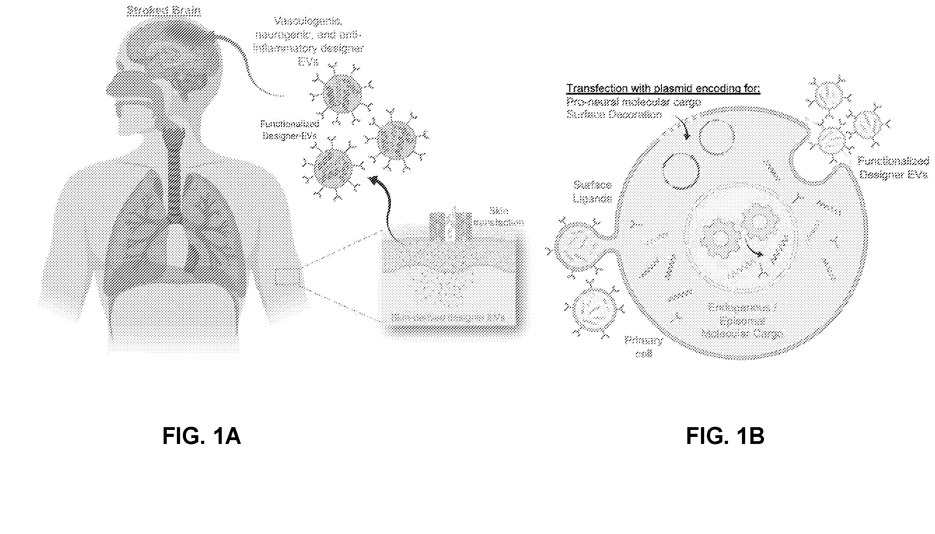

FIGs. 1A and 1B illustrate nanoscale engineering of designer extracellular vesicles for therapeutic applications.

| Table 1. Examples of molecular cargo and mechanism of action of designer EV therapies for ischemic stroke | | |
|---|---|---|
| Proposed Therapy | Plasmids | Rationale |
| Designer EVs loaded with proneurogenic factors | ASCL1, BRN2, and MYT1L (ABM) | • Shown to promote neuronal phenotype¹ |
| | | • May induce direct cell reprogramming of resident/ scaring glia into neurons at the infarcted area, helping to repair damaged nerve tissue |
| Designer EVs loaded with proangiogenic factors | FOXC2, ETV2, or FLI1 | • Shown to promote vasculogenesis in vivo^{2,4} |
| | | • May induce direct cell reprogramming of resident/ scaring glia into functionalized endothelium at the infarcted area, ² helping to re-establish proper tissue perfusion |
| Designer EVs loaded with anti-inflammatory cargo | IL-4 and IL-10 | • IL-4 is secreted by ischemic neurons to favor an anti-inflammatory phenotype and increased phagocytic activity in microglia.⁵ |
| | | • IL-10 has the potential to reduce infarct size when administered to the lateral ventricle after stroke.⁶ |
| 1. Pfisterer, U. et al. PNAS 2011 108: 10343-48. | | |
| 2. Gallego-Perez, D, et al. Nat Nanotechnol 2017 12:974-979 | | |
| 3. Chanda, S, et al. Stem Cell Reports 2014 3:282-296 | | |
| 4. Mnorita, R, et al. Proc Natl Acad Sci USA 2015 112:160-165 | | |
| 5. Zhao, X, et al. J Neurosci 2015 11281-11291 | | |
| 6. Doll, DN, et al. Aging Dis 2014 5:294-306 | | |

FIGs. 2A to 2E show proneural designer EVs characterization. FIG. 2A shows transmission electron micrograph of designer EVs derived from primary mouse embryonic fibroblasts (PMEFs) and loaded with the proneural factors the ASCL1, BRN2, and MYT1L (i.e., ABM cocktail). FIG. 2B shows dynamic of proneural designer EV release showing a peak 24 hours after nanotransfection of PMEF, with a particle concentration in the order of ten billion particles per mL (*p-value=0.018). FIG. 2C shows the number of gene copies packed inside these proneural designer EVs is approx. 3 orders of magnitude higher than that delivered to the donor cell for all three factors (*p-value=0.021). FIG. 2D shows proneural designer EVs are successfully captured by PMEFs in culture, with a peak in uptake 48 hours after treatment (*p-value ≤ 0.036). FIG. 2E Fluorescent image of PMEF cells incorporating fluorescently labeled (red) proneurogeninc-EVs 24 hours after treatment.

FIG. 3A shows confocal images of donor cells transfected with the mGluR8 or a sham vector, showing positive colocalization of cell membrane (green and white) and the transfected glutamate receptor (red) only for mGluR8 transfected cells. FIGs. 3B and 3C show Confocal images of EVs derived from sham or mGluR8 transfected donor cells, where EVs derived from GlutR8 transfected cells show co-localization of the EV membrane (green) with the targeting receptor (red). FIG. 3D Western blot of mGluR8-functionalized EVs showing positive protein expression compared to sham (control)-EVs.

FIGs. 4A to 4C show functionalized designer EV to target the brain. FIG. 4A shows in vivo imaging of brains after intranasal delivery of fluorescently labeled designer EVs functionalized with mGluR8 or sham (control) EVs, showing significantly higher accumulation of functionalized EVs in the brain 24 hours after treatment. FIGs. 4B and 4C show immunofluorescence images of cerebellum and corpus callosum (sagittal cut) of brains of animals treated with fluorescently labeled (red) sham- or mGluR8-EVs, 24 hours post-intranasal instillation, and respective fluorescence intensity quantification (n=3, *p-value=0.0083).

### Example 2:

FIGs. 3E and 3F show characterization of designer EVs functionalized with mGRM4 and mGRM8 derived from PMEFs 24 hours after nanotransfection with plasmids encoding for each receptor or sham vector, with a particle concentration in the range of billions of EVs per mL and an average particle approximately 230 nm.

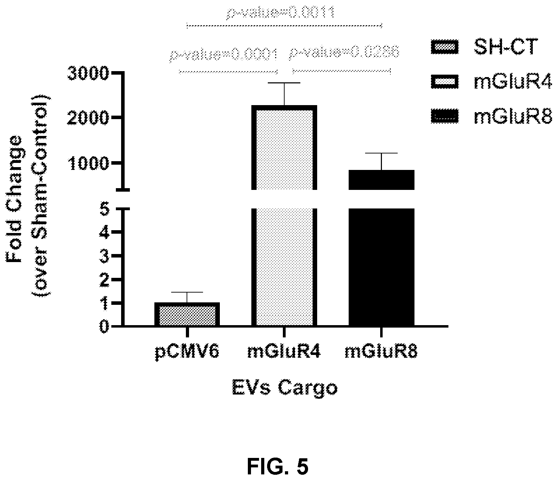

FIG. 5 show characterization of relative expression of metabotropic glutamate receptors (mGluR4 and mGluR8) in designer EVs, these receptors are used to functionalize neurogenic designer EVs.

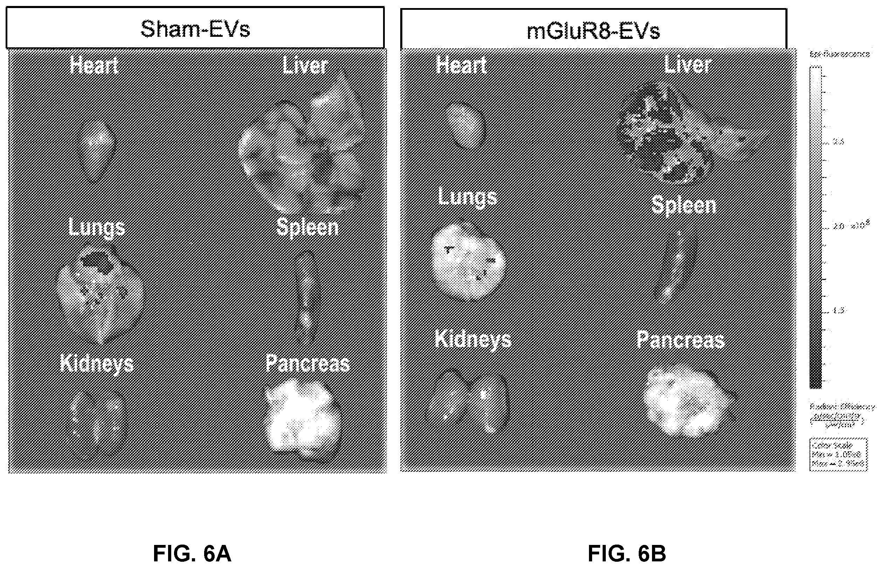

FIGs. 6A and 6B show designer EV biodistribution 24 hours after intranasal delivery showing higher accumulation in the brain of mice treated with designer EVs functionalized with mGluR8 vs. non-functionalized designer EVs, which accumulate in the liver tissue as they are cleared from the body.

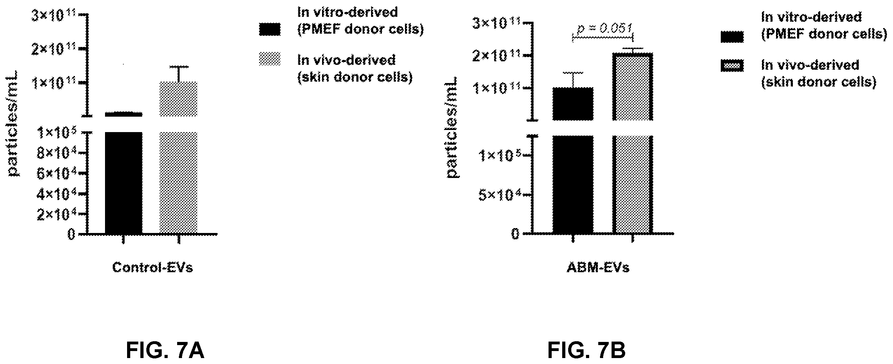

FIGs 7A and 7B show comparison of yield for in vitro-derived (using PMEF as donor cells) vs. in vivo-derived (using skin cells as donor cells) ABM- and control-designer EVs, showing that a significantly higher number of EVs are produced in vivo.

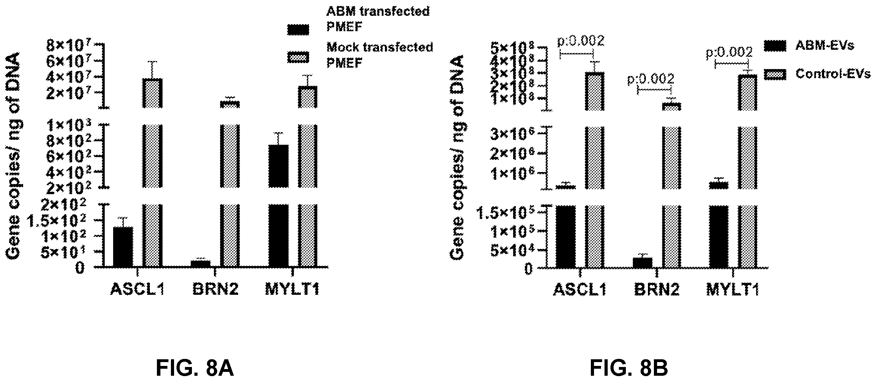

FIGs. 8A and 8B show efficiency of molecular loading of neurogenic factors ACL1, BRN2, and MYT1L (ABM) inside designer EVs vs. number of gene copies inside donor cells 24 hours after transfection.

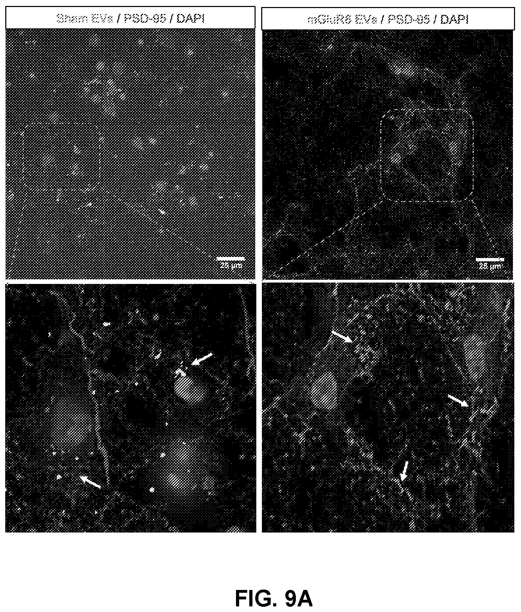

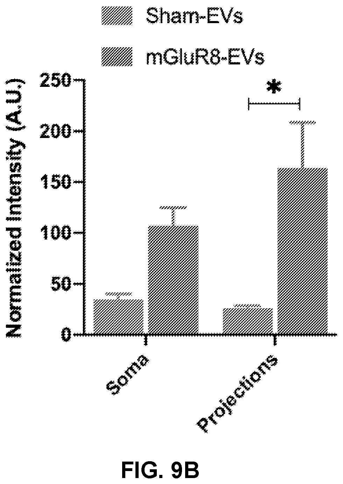

FIG. 9A shows immunofluorescence images of primary neurons incorporating mGluR8 functionalized EVs (red) and control-EVs (green), showing preferential accumulation of GluR-8 EVs in postsynaptic regions (postsynaptic protein staining, PSD-95) (violet), with zoom-in regions for each type of sample (bottom) 7hours after treatment. FIG. 9B shows quantification of green (sham-EVs) or red (mGluR8-EVs.

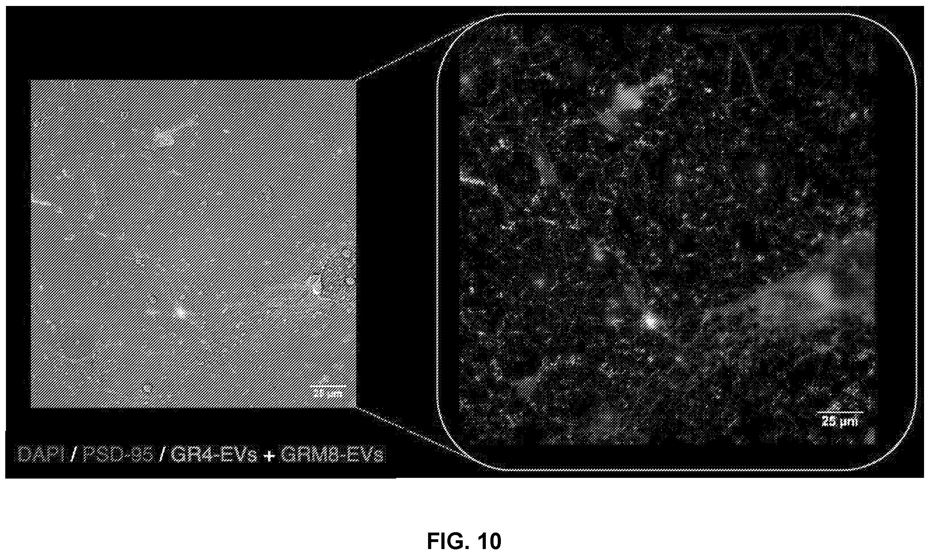

FIG. 10 shows mGluR4- and mGLuR8-functionalized designer EVs uptake by primary mouse embryonic neurons 8 hours after treatment.

FIG. 11A shows how prolonged culture studies suggest that PMEFs exposed to ABM-loaded EVs exhibit pro-neuronal conversions, as evidence by the increased in immunoreactivity for Tuj1 (green), a neuronal marker, relative to PMEFs exposed to control EVs as early as 7 days after treatment. These data suggest that the extent of plasmid DNA transfer from EVs to recipient cells falls within the same order of magnitude compared to direct electroporation. Additionally, the induction of Tuj1 immunoreactivity in fibroblast cultures suggest that ABM-loaded EVs could potentially be used to drive pro-neuronal responses/conversions in non-neuronal cells. FIG. 11B shows the quantification of Tuj1 fluorescence intensity 7- and 14-days post-treatment (n=3, *p-value=0.043, **p-value=0.004). Unless defined otherwise, all technical and scientific terms used herein have the same meanings as commonly understood by one of skill in the art to which the disclosed invention belongs.

Those skilled in the art will recognize, or be able to ascertain using no more than routine experimentation, many equivalents to the specific embodiments of the invention described herein.

## Claims

1. A composition comprising extracellular vesicles (EVs) produced from donor cells engineered to express a glutamate receptor (GluR), wherein the GluR is a metabotropic glutamate receptor-4 (GRM4) or a metabotropic glutamate receptor-8 (GRM8).

2. The composition of claim 1, wherein the donor cells are skin cells.

3. The composition of claim 1 or 2, wherein the EVs encapsulate a therapeutic or diagnostic cargo.

4. The composition of claim 3, wherein the therapeutic cargo comprises a proangiogenic, proneurogenic, or anti-infalmmatory molecular cargo.

5. The composition of claim 3, wherein the diagnostic cargo comprises a molecular beacon.

6. The composition of any of claims 3 to 4 for use in the treatment of a CNS injury resulting in excitotoxicity in a subject, comprising administering to the subject an effective amount of the composition of any one of claims 3 to 4 wherein the EVs encapsulate a therapeutic cargo and wherein the CNS injury resulting in excitotoxicity is selected from the group consisting of spinal cord injury, stroke, traumatic brain injury and a neurodegenerative disease.

7. The composition for use of claim 6, wherein the neurodegenerative disease is Alzheimer's disease, Parkinson's disease, a Parkinson's-related disorder, Huntington's disease, prion disease, motor neuron disease (MND), spinocerebellar ataxia (SCA) or spinal muscular atrophy (SMA).

## Patentansprüche

1. Eine Zusammensetzung, die aus Spenderzellen hergestellte extrazelluläre Vesikel (EV) umfasst, die verändert wurden, um einen Glutamatrezeptor (GIuR) zu exprimieren, wobei der GluR ein metabotroper Glutamatrezeptor-4 (GRM4) oder ein metabotroper Glutamatrezeptor-8 (GRM8) ist.

2. Die Zusammensetzung nach Anspruch 1, wobei die Spenderzellen Hautzellen sind.

3. Die Zusammensetzung nach Anspruch 1 oder 2, wobei die EV eine therapeutische oder diagnostische Fracht enthalten.

4. Die Zusammensetzung nach Anspruch 3, wobei die therapeutische Fracht eine proangiogene, proneurogene oder entzündungshemmende molekulare Fracht umfasst.

5. Die Zusammensetzung nach Anspruch 3, wobei die diagnostische Fracht ein molekulares Leuchtsignal (molecular beacon) umfasst.

6. Die Zusammensetzung nach einem der Ansprüche 3 bis 4 zur Verwendung bei der Behandlung von einer Verletzung des Zentralnervensystems (ZNS), welche zu Exzitotoxizität in einem Subjekt führt, umfassend die Verabreichung einer wirksamen Menge der Zusammensetzung nach einem der Ansprüche 3 bis 4 an das Subjekt,
wobei die EV eine therapeutische Fracht verkapseln und wobei die ZNS-Verletzung, welche zu Exzitotoxizität führt, aus der Gruppe ausgewählt ist, die aus Rückenmarksverletzung, Schlaganfall, traumatische Hirnverletzung und neurodegenerativer Erkrankung besteht.

7. Die Zusammensetzung zur Verwendung nach Anspruch 6, wobei die neurodegenerative Erkrankung Alzheimer, Parkinson, eine Parkinson-ähnliche Erkrankung, Huntington, eine Prionenerkrankung, eine Motoneuronerkrankung (MND), spinozerebelläre Ataxie (SCA) oder eine spinale Muskelatrophie (SMA) ist.

## Revendications

1. Composition comprenant des vésicules extracellulaires (EV) produits à partir de cellules donneuses modifiées pour exprimer un récepteur du glutamate (GluR), dans lequel le GluR est un récepteur métabotropique du glutamate 4 (GRM4) ou un récepteur métabotropique du glutamate 8 (GRM8).

2. Composition selon la revendication 1, dans laquelle les cellules donneuses sont des cellules de peau.

3. Composition selon la revendication 1 ou 2, dans laquelle les EV encapsulent une cargaison thérapeutique ou diagnostique.

4. Composition selon la revendication 3, dans laquelle la cargaison thérapeutique comprend une cargaison moléculaire pro-angiogénique, pro-neurogène ou anti-inflammatoire.

5. Composition selon la revendication 3, dans laquelle la cargaison diagnostique comprend une balise moléculaire.

6. Composition selon l'une quelconque des revendications 3 à 4 pour une utilisation dans le traitement d'une lésion du SNC résultant en une excitotoxicité chez un sujet, comprenant l'administration au sujet d'une quantité active de la composition selon l'une quelconque des revendications 3 à 4 dans laquelle les EV encapsulent une cargaison thérapeutique et dans laquelle la lésion du SNC résultant en une excitotoxicité est choisie dans le groupe constitué par une lésion de la moelle épinière, un accident vasculaire cérébral, une lésion cérébrale traumatique et une maladie neurodégénérative.

7. Composition pour une utilisation selon la revendication 6, dans laquelle la maladie neurodégénérative est la maladie d'Alzheimer, la maladie de Parkinson, un trouble lié à Parkinson, la chorée de Huntington, une maladie à prions, une maladie du motoneurone (MND), l'ataxie spinocérébelleuse (SCA) ou l'amyotrophie spinale (SMA).
